(19) Europäisches Patentamt
European Patent Office
Office européen des brevets

(11) EP 1 200 402 B1

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(21) Anmeldenummer: **00956273.7**

(22) Anmeldetag: **25.07.2000**

(51) Int Cl.[7]: **C07D 207/44**, A61K 31/40, A61P 29/00

(86) Internationale Anmeldenummer:
**PCT/EP2000/007100**

(87) Internationale Veröffentlichungsnummer:
**WO 2001/010833 (15.02.2001 Gazette 2001/07)**

(54) **SUBSTITUIERTE 1,5-DIHYDROPYRROL-2-ON-DERIVATE WIRKSAM ALS NMDA-REZEPTOR-ANTAGONISTEN FÜR DIE BEHANDLUNG VON SCHMERZZUSTÄNDEN**

SUBSTITUTED 1,5-DIHYDROPYRROL-2-ON DERIVATIVES AS NMDA RECEPTOR ANTAGONISTS FOR THE TREATMENT OF PAIN

DERIVES SUBSTITUES DE 1,5-DIHYDROPYRROL-2-ONE EN TANT QU'ANTAGONISTES DU RECEPTEUR NMDA POUR TRAITER LA DOULEUR

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **06.08.1999 DE 19936719**

(43) Veröffentlichungstag der Anmeldung:
**02.05.2002 Patentblatt 2002/18**

(73) Patentinhaber: **Grünenthal GmbH**
**52078 Aachen (DE)**

(72) Erfinder:
- **PRZEWOSNY, Michael**
  **D-52062 Aachen (DE)**
- **STACHEL, Hans-Dietrich**
  **D-82061 Neuried (DE)**
- **POSCHENRIEDER, Hermann**
  **D-82166 Gräfelfing (DE)**

(56) Entgegenhaltungen:
**WO-A-86/01716**
**GB-A- 2 170 498**
**US-A- 5 420 155**

- **POSCHENRIEDER ET AL: "5-Aryliden-pyrrolidine-2,3,4-trione 3 Oximes as NMDA Receptor Antagonist" ARCHIV DER PHARMAZIE,VCH VERLAGSGESELLSCHAFT MBH, WEINHEIM,DE, Bd. 332, Nr. 9, 1999, Seiten 309-316, XP002146311 ISSN: 0365-6233**
- **DATABASE CAOLD [Online] CHEMICAL ABSTRACTS SERVICE, COLUMBUS, OHIO, US; SCHMIDT, ULRICH ET AL: "total synthesis of the antibiotics thiolutin and holomycin" retrieved from STN Database accession no. 6374h XP002154797**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte 1,5-Dihydropyrrol-2-on-Derivate, Verfahren zu deren Herstellung, Arzneimittel enthaltend diese Verbindungen sowie die Verwendung dieser Verbindungen zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem zeigen sie bei einigen Schmerzzuständen, insbesondere bei neuropathischen und inzidentiellen Schmerzen, eine geringere Wirksamkeit.

Opioide entfalten ihre analgetische Wirkung durch Bindung an membranständige Rezeptoren, die zur Familie der sogenannten G-Proteingekoppelten Rezeptoren gehören. Daneben gibt es weitere Rezeptoren und Ionenkanäle, die wesentlich an dem System der Schmerzentstehung und der Schmerzweiterleitung beteiligt sind, wie z.B. der N-Methyl-D-Aspartat-(NMDA)-Ionenkanal, über den ein wesentlicher Teil der Kommunikation von Synapsen abläuft und durch den der Calcium-Ionenaustausch zwischen einer neuronalen Zelle und ihrer Umgebung gesteuert wird.

Kenntnisse über die physiologische Bedeutung von Ionenkanal-selektiven Substanzen sind durch die Entwicklung der patch-clamp-Technik gewonnen worden, mit der sich die Wirkung von NMDA-Antagonisten auf den Calciumhaushalt im Zellinneren nachweisen läßt.

Aus GB 2 170 498 ist ein Verfahren zur Herstellung von Dithiolopyrrolonen mit insektiziden Eigenschaften bekannt.

Der Datenbankauszug CA59:6374h offenbart die Verbindung 3-Acetamido-5-(ethoxymethylen)-4-hydroxy-1-methyl-3-pyrrolin-2-on-acetat.

Der WO 86/01716 sind Pyrrothinderivate mit antiallergischer Wirkung zu entnehmen.

Die US 5,420,155 beschreibt Tetramsäure-Derivate mit NMDA-antagonistischer Wirkung.

Der nach dem Prioritätstag dieser Anmeldung veröffentlichten Literaturveröffentlichung von Stachel et al. in Arch. Pharm. Pharm. Med. Chem., 1999, 332, 309-316 sind 5-Aryliden-pyrrolidin-2,3,4-trion-3-oxime und deren Eignung zur Verwendung als NMDA-Rezeptor-Antagonisten zu entnehmen.

Eine der Erfindung zugrundeliegende Aufgabe bestand in dem zur Verfügung stellen von neuen Verbindungen, die sich zur Schmerztherapie oder zur Anxiolyse eignen. Darüber hinaus sollten diese Verbindungen möglichst wenig Nebenwirkungen der Opioid-Analgetika wie z.B. Übelkeit, Erbrechen, Abhängigkeit, Atemdepression oder Obstipation aufweisen. Weitere Aufgaben bestanden darin, neue Wirkstoffe zur Behandlung von inflammatorischen und/oder allergischen Reaktionen, Depressionen, Drogen- und/oder Alkoholmißbrauch, Gastritis, Diarrhoe, Haminkontinenz, cardiovaskulären Erkrankungen, Atemwegserkrankungen, Husten, seelischen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Himödemen, Hypoxie, Anoxie, AIDS-Demens, Encephalomyelitis, Tourette-Syndrom, Tinnitus aurium oder perinataler Asphyxie zur Verfügung zu stellen.

Es wurde nun gefunden, daß substituierte 1,5-Dihydropyrrol-2-on-Derivate der nachstehenden allgemeinen Formel I als NMDA-Antagonisten selektiv an der Glycin-Bindungsstelle angreifen und sich zur Behandlung von inflammatorischen und/oder allergischen Reaktionen, Depressionen; Drogen- und/oder Alkoholmißbrauch, Gastritis, Diarrhoe, Haminkontinenz, cardiovaskulären Erkrankungen, Atemwegserkrankungen, Husten, seelischen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Himödemen, Hypoxie, Anoxie, AIDS-Demens, Encephalomyelitis, Tourette-Syndrom, Tinnitus aurium oder perinataler Asphyxie eignen und die außerdem eine ausgeprägte analgetische und/oder anxiolytische Wirkung aufweisen.

Gegenstand der vorliegenden Erfindung sind daher substituierte 1,5-Dihydropyrrol-2-on-Derivate der allgemeinen Formel I,

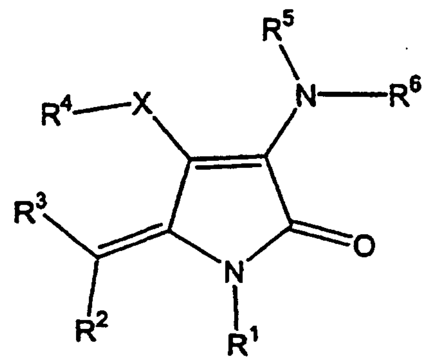

I

worin
X für O, oder $NR^7$ steht,
der Rest $R^1$ für H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe, vorzugsweise für einen über eine $C_{1-3}$-Alkylen-Gruppe gebundenen Aryl-Rest steht,
die Reste $R^2$, $R^3$, gleich oder verschieden, für H, F, Cl, Br, $CF_3$, $OR^{11}$, $SR^{11}$, $NR^9R^{10}$, einen $C_{1-10}$-Alkyl-, vorzugsweise für einen $C_{1-6}$-Alkyl-, einen Aryl-, einen Heteroaryl-Rest oder für einen über eine $C_{1-6}$-Alkylen-Gruppe, vorzugsweise für einen über eine $C_{1-3}$-Alkylen-Gruppe gebundenen Aryl-Rest stehen,
der Rest $R^4$ für H, OH, $OR^{11}$, $SR^{11}$, $COR^8$, $COOR^8$, $COCOR^8$, $CONR^9R^{10}$, $CSNR^9R^{10}$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,
die Reste $R^5$, $R^6$, gleich oder verschieden, für H, O, OH, $OR^{11}$, $SR^{11}$, $COR^8$, $CSR^8$, $COOR^8$, $COCOR^8$, $CONR^9R^{10}$, $CSNR^9R^{10}$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest stehen oder $R^5$ und $R^6$ zusammen die Gruppe =O bedeuten,
der Rest $R^7$ für H, $OR^{11}$, $SR^{11}$, $COR^8$, $COOR^8$, $COCOR^8$, $CONR^9R^{10}$, $CSNR^9R^{10}$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,
der Rest $R^8$ für H, $OR^{11}$, $SR^{11}$, $NR^9R^{10}$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,
der Rest $R^9$ für einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,
der Rest $R^{10}$ für einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,
der Rest $R^{11}$ für einen $C_{1-10}$-Alkyl-, vorzugsweise einen $C_{1-6}$-Alkyl-, einen Aryl-, einen Heteroaryl- oder für einen über eine $C_{1-6}$-Alkylen-Groppe gebundenen, vorzugsweise für einen über eine $C_{1-3}$-Alkylen-Gruppe gebundenen Aryl-Rest steht,
in Form ihrer Racemate, Enantiomere, Diastereomere oder einer entsprechenden Base oder eines entsprechenden physiologisch verträglichen Salzes,
wobei die Verbindung 3-Acetamido-5-(ethoxy methylen)-4-hydroxy-1-methyl-3-pyrrolin-2-on-acetat sowie die Racemate
der 1-Methyl-3-acetamino-5-ethoxymethylentetramsäure,
der 3-Acetamino-5-ethoxymethylentetramsäure und
der $\alpha$-acetylamino-$\gamma$-benzylthiomethylentetramsäure
ausgenommen sind.

Unter Alkyl-Resten werden auch verzweigte, unverzweigte oder cyclische unsubstituierte oder mindestens einfach, vorzugsweise mit F, Cl, Br, CN, $NO_2$, CHO, $SO_2C_{1-6}$-Alkyl, $SO_2CF_3$, $OR^8$, $NR^9R^{10}$, $COR^8$, $COOR^8$, $COCOR^8$,

$CONNR^9R^{10}$ und/oder $CSNR^9R^{10}$ substituierte Kohlenwasserstoffe verstanden, wobei die Reste $R^8$ bis $R^{10}$ die Bedeutung gemäß der allgemeinen Formel I haben. Enthalten die Alkyl-Reste mehr als einen Substituenten, so können diese gleich oder verschieden sein. Vorzugsweise sind die Alkylreste Methyl, Ethyl, Propyl, Isopropyl, n-Butyl, sek-Butyl, tert-Butyl, Neopentyl, n-Hexyl, Cyclopropyl, Cyclobutyl, Cyclopentyl oder Cyclohexyl.

Unter einem Aryl-Rest werden auch unsubstituierte oder mindestens einfach mit OH, F, Cl, Br, $CF_3$, CN, $NO_2$, CHO, $SO_2C_{1-6}$-Alkyl, $SO_2CF_3$, $NR^9R^{10}$, $COR^8$, $COOR^8$, $COCOR^8$, $CONR^9R^{10}$, $CSNR^9R^{10}$, einem $C_{1-6}$-Alkyl-Rest, einem $C_{1-6}$-Alkoxy-Rest, einem $C_{2-6}$-Alkylen-Rest, einem Heterocyclyl-Rest und/oder einem Phenyl-Rest substituierte Phenyle verstanden, wobei die Reste $R^8$ bis $R^{10}$ die Bedeutung gemäß der allgemeinen Formel I haben. Enthalten die Aryl-Reste mehr als einen Substituenten, so können diese gleich oder verschieden sein. Der Ausdruck kann auch Naphthyl bedeuten. Die Phenylreste können auch mit weiteren Ringen kondensiert sein.

Unter einem Heteroaryl-Rest werden auch 5- oder 6-gliedrige ungesättigte, gegebenenfalls mit einem ankondensierten Aryl-Rest versehene, heterocyclische Verbindungen verstanden, die wenigstens ein Heteroatom, vorzugsweise Stickstoff und/oder Sauerstoff und/oder Schwefel enthalten. Vorzugsweise ist der Heteroaryl-Rest Furan, Thiophen, Pyrrol, Pyridin, Pyrimidin, Chinolin, Isochinolin, Phthalazin oder Chinazolin.

Besonders bevorzugt sind folgende substituierte 1,5-Dihydropyrrol-2-on-Derivate:

5-Benzyliden-4-methoxy-3-nitroso-1,5-dihydropyrrol-2-on,

4-Benzylamin-5-benzyliden-3-nitroso-1,5-dihydropyrrol-2-on,

5-Benzyliden-4-hydroxy-3-nitroso-1,5-dihydropyrrol-2-on,

5-Benzyliden-3-nitroso-4-phenylamino-1,5-dihydropyrrol-2-on,

5-Benzyliden-4-methylamino-3-nitroso-1,5-dihydropyrrol-2-on,

4-Amino-5-benzyliden-3-nitroso-1,5-dihydropyrrol-2-on,

5-Benzyliden-3-nitro-4-phenylamino-1,5-dihydropyrrol-2-on,

4-Benzylamino-5-benzyliden-3-nitro-1,5-dihydropyrrol-2-on,

5-Benzyliden-4-methylamino-3-nitro-1,5-dihydropyrrol-2-on
und

4-Amino-5-benzyliden-3-nitro-1,5-dihydropyrrol-2-on.

Ein weiterer Gegenstand der Erfindung sind Verfahren zur Herstellung von substituierten 1,5-Dihydropyrrol-2-on-Derivaten der allgemeinen Formel I, in denen man Tetramsäuren der allgemeinen Formel II

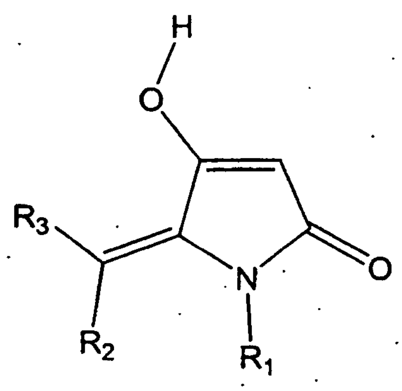

II

worin die Reste $R^1$ bis $R^3$ die Bedeutung gemäß der allgemeinen Formel I haben, bei niedriger Temperatur in Lösung, vorzugsweise in eisgekühlter, saurer Lösung, besonders bevorzugt in eisgekühlter, saurer Lösung von Eisessig, mit einer wäßrigen Lösung von Alkalinitrit, vorzugsweise Natriumnitrit zu Verbindungen der allgemeinen Formel III,

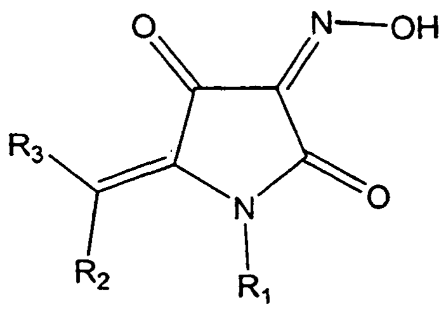

III

worin die Reste $R^1$ bis $R^3$ die Bedeutung gemäß der allgemeinen Formel I haben, umsetzt, und diese vorzugsweise durch Umkristallisation, bevorzugt aus Ethanol reinigt und isoliert. Diese Verbindungen der allgemeinen Formel III liegen im allgemeinen im Gemisch mit den entsprechenden Nitroso-Tautomeren vor.

Die Synthese der Ausgangsverbindungen, der Tetramsäuren der allgemeinen Formel II, kann nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696) sowie den darin zitierten Literaturstellen durchgeführt werden. Sie werden hiermit als Referenz eingeführt und gelten somit als Teil der Offenbarung.

Die Verbindungen der allgemeinen Formel III, worin $R^1$ bis $R^3$ die Bedeutung gemäß der allgemeinen Formel I haben, können durch Alkylierung mit an sich bekannten Alkylierungsmitteln, vorzugsweise mit Diazoalkanen, Dialkylsulfaten oder Alkylhalogeniden, in Lösung, besonders bevorzugt mit Diazoalkanen in etherischer Lösung oder durch die Umsetzung mit Säurechloriden, Säurebromiden, Chlorkohlensäureestern, Fluorkohlensäureestern, Isocyanaten oder Isothiocyanaten in unpolaren Lösungsmitteln, vorzugsweise in offenkettigen oder cyclischen Ethern, Kohlenwasserstoffen, halogenhaltigen Kohlenwasserstoffen, und/oder in polaren, aprotischen Lösungsmitteln, vorzugsweise in Dimethylformamid und/oder N-Methylpyrrolidon, und/oder in polaren, protischen Lösungsmitteln, vorzugsweise Dimethylsulfoxid, zu Verbindungen der allgemeinen Formel IV

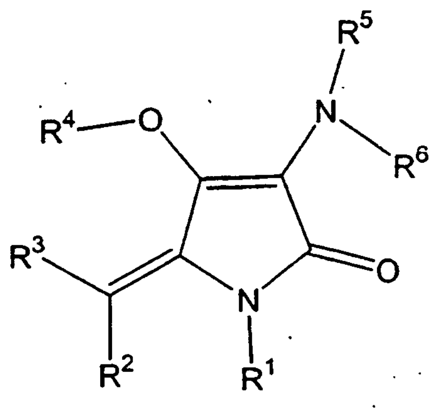

IV

umgesetzt werden, worin die Reste $R^1$ bis $R^4$ die Bedeutung gemäß der allgemeinen Formel I haben und die Reste $R^5$ und $R^6$ zusammen die Gruppe =O bedeuten, die nach den üblichen Methoden gereinigt und isoliert werden.

Die Verbindungen der allgemeinen Formel IV, worin die Reste $R^1$ bis $R^4$ die Bedeutung gemäß der allgemeinen Formel I haben und die Reste $R^5$ und $R^6$ zusammen die Gruppe =O bedeuten, können noch weiter derivatisiert werden, in dem sie mit Nukleophilen, vorzugsweise mit primären oder sekundären Aminen, aliphatischen, aromatischen oder heteroaromatischen Alkoholaten oder entsprechende Thiolaten, Phenolaten und/oder Thiophenolaten in polaren Lösungsmitteln, vorzugsweise in Methanol, Ethanol und/oder Isopropanol zu Verbindungen der allgemeinen Formel V

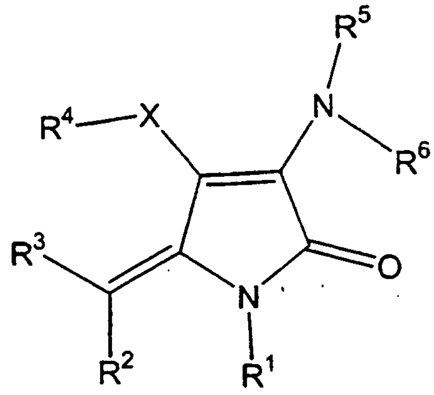

V

umgesetzt werden, worin die Reste $R^1$ bis $R^4$ sowie X die Bedeutung gemäß der allgemeinen Formel I haben und die Reste $R^5$ und $R^6$ zusammen die Gruppe =O bedeuten, durch Umkristallisation, vorzugsweise durch Umkristallisation aus Methanol, Ethanol und/oder Isopropanol gereinigt und isoliert werden.

Die Verbindungen der allgemeinen Formel V, worin die Reste $R^1$ bis $R^4$ sowie X die Bedeutung gemäß der allgemeinen Formel I haben und die Reste $R^5$ und $R^6$ zusammen die Gruppe =O bedeuten, können zu Verbindungen der allgemeinen Formel I, worin die Reste $R^5$ und $R^6$ jeweils H bedeuten und $R^1$ bis $R^4$ sowie X die Bedeutung gemäß der allgemeinen Formel I haben, durch Umsetzung mit einem Reduktionsmittel, vorzugsweise mit Zn/Eisessig, Lithiumaluminiumhydrid, $BH_3$, $Na[BH_3CN]/TiCl_3$, Natriumalkoxiden oder durch Hydrierung mit Wasserstoff in Gegenwart von Übergangsmetallkatalysatoren reduziert werden.

Eine weitere Derivatisierung der Verbindungen der allgemeinen Formel I, in denen $R^5$ und $R^6$ jeweils H bedeuten und die Reste $R^1$ bis $R^4$ sowie X die Bedeutung gemäß der allgemeinen Formel I haben, zu Verbindungen der allgemeinen Formel I, worin die Reste $R^5$, $R^6$, gleich oder verschieden, für O, OH, $OR^{11}$, $SR^{11}$, $COR^8$, $CSR^8$, $COOR^8$, $COCOR^8$, $CONR^9R^{10}$, $CSNR^9R^{10}$, einen $C_{1-10}$-Alkyl-Rest, einen Aryl-Rest, einen Heteroaryl-Rest oder für einen über eine $C_{1-6}$-Alkylen-Gruppe gebundenen Aryl-Rest stehen und die Reste $R^1$ bis $R^4$ sowie X die Bedeutung gemäß der allgemeinen Formel I haben, kann nach verschiedenen, dem Fachmann bekannten Methoden erfolgen.

Die Verbindungen der allgemeinen Formel V, worin $R^5$ und $R^6$ zusammen die Gruppe =O bedeuten, können auch durch Oxidation der Verbindungen der allgemeinen Formel III, vorzugsweise mit $KMnO_4$ und Peroxytrifluoressigsäure erhalten und nach den üblichen Verfahren gereinigt und isoliert werden.

Die erfindungsgemäßen Verbindungen der allgemeinen Formel I lassen sich mit Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in die entsprechenden physiolgisch verträglichen Salze überführen. Vorzugsweise wird die Salzbildung in einem Lösungsmittel, wie beispielsweise Diethylether, Diisopropylether, Essigsäurealkylester, Aceton und/oder 2-Butanon durchgeführt. Zur Herstellung der entsprechenden Hydrochloride eignet sich darüber hinaus Trimeihylchlorsilan in wäßriger Lösung.

Die erfindungsgemäßen substituierten 1,5-Dihydropyrrol-2-on-Derivate der allgemeinen Formel Ia gemäß Anspruch 30 sind toxikologisch unbedenklich und stellen daher geeignete pharmazeutische Wirkstoffe dar.

Ein weiterer Gegenstand der Erfindung sind daher Arzneimittel, die als Wirkstoff wenigstens ein substituiertes 1,5-Dihydropyrrol-2-on-Derivat der allgemeinen Formel Ia und/oder eine entsprechende Base und/oder ein entsprechendes physiologisch verträgliches Salz sowie gegebenenfalls weitere Wirk- und/oder Hilfsstoffe enthalten. Das Arzneimittel kann auch ein Gemisch aus wenigstens zwei Enantiomeren und/oder entsprechender Basen und/oder entsprechender physiologisch verträglicher Salze einer erfindungsgemäßen Verbindung der allgemeinen Formel Ia enthalten, wobei die Enantiomeren nicht in äquimolaren Gemischen vorliegen.

Vorzugsweise werden die Arzneimittel zur Bekämpfung von Schmerzen oder zur Behandlung von inflammatorischen und/oder allergischen Reaktionen, Depressionen, Drogen- und/oder Alkoholmißbrauch, Gastritis, Diarrhoe,

Harninkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Himödemen, Unterversorgungszuständen des zentralen Nervensystems, Hypoxie, Anoxie, AIDS-Demens, Encephalomyelitis, Tourette-Syndrom, perinataler Asphyxie, Tinnitus aurium oder zur Anxiolyse eingesetzt.

Ein weiterer Gegenstand der Erfindung ist auch die Verwendung wenigstens eines substituierten 1,5-Dihydropyrrol-2-on-Derivates der allgemeinen Formel Ia und/oder einer entsprechenden Base und/oder eines entsprechenden physiologisch verträglichen Salzes zur Herstellung eines Arzneimittels zur Bekämpfung von Schmerzen und/oder zur Behandlung von inflammatorischen und/oder allergischen Reaktionen, Depressionen, Drogen- und/oder Alkoholmißbrauch, Gastritis, Diarrhoe, Haminkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Himödemen, Unterversorgungszuständen des zentralen Nervensystems, Hypoxie, Anoxie, AIDS-Demens, Encephalomyelitis, Tourette-Syndrom, perinataler Asphyxie, Tinnitus aurium oder zur Anxiolyse.

Zur Zubereitung entsprechender pharmazeutischer Formulierungen können neben mindestens einem substituierten 1,5-Dihydropyrrol-2-on-Derivat der allgemeinen Formel Ia Hilfsstoffe, wie z.B. Trägermaterialien, Füllstoffe, Lösungsmittel, Verdünnungsmittel, Farbstoffe und/oder Bindemittel eingesetzt werden. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Tropfen, Säften und Sirupen sowie multipartikuläre Zubereitungen, beispielsweise Pellets oder Granulate, die ggf. auch in Kapseln abgefüllt oder zu Tabletten verpreßt sein können, für die parenterale, topische und inhalative Applikation Lösungen, Suspensionen, leicht rekonstituierbare Trockenzubereitungen sowie Sprays. Erfindungsgemäße Verbindungen der allgemeinen Formel Ia in einem Depot, in gelöster Form oder in einem Pflaster, gegebenenfalls unter Zusatz von die Hautpenetration fördemden Mitteln, sind geeignete perkutane Applikationszubereitungen. Oral oder perkutan anwendbare Zubereitungsformen können die erfindungsgemäßen Verbindungen der allgemeinen Formel Ia auch verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 2 bis 500 mg/kg Körpergewicht des Patienten wenigstens eines substituierten 1,5-Dihydropyrrol-2-on-Derivates der allgemeinen Formel Ia appliziert.

Pharmakologische Untersuchungen:

a) Untersuchungen zur Rezeptorbindung

Die Untersuchungen zur Bestimmung der Affinität der erfindungsgemäßen substituierten 1,5-Dihydropyrrol-2-on-Derivate der allgemeinen Formel Ia zur Glycin-Bindungsstelle des NMDA-Rezeptorkanals wurde an Himmembranhomogenaten (Homogenat von Cortex- und Hippocampus-Areal aus dem Hirn von männlichen Ratten, Stamm Wistar, Charles River, WIGA GmbH, Sulzbach, Deutschland) nach Baron B.M. et al, J. Pharmacol. Exp. Ther., Vol. 279, pp. 62-68 (1996) durchgeführt.

Hierzu wurde Cortex und Hippocampus aus frisch entnommenen Rattengehirnen freipräpariert und in 5 mmol/l TRIS-Acetatpuffer, 0,32 mol/l Sacharose pH 7,4 (10 ml/g Frischgewicht) mit einem Potter-Homogenisator (Fa. Braun, Melsungen, Deutschland, 10 Kolbenhübe bei 500 Umdrehungen pro Minute (Upm)) unter Eiskühlung homogenisiert und anschließend für 10 Minuten bei 1.000 g und 4°C zentrifugiert. Der erste Überstand wurde gesammelt und das Sediment erneut mit 5 mmol/l TRIS-Acetatpuffer, 0,32 mol/l Sacharose pH 7,4 (5 ml/g ursprüngliche Frischeinwaage Rattenhim-Cortex und Hippocampus) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wurde das Membranhomogenat für 1 Stunde bei 4°C inkubiert und für 30 Minuten bei 50.000 g und 4°C zentrifugiert. Der Überstand wurde verworfen und das Zentrifugenröhrchen mit dem Membransediment mit Parafilm verschlossen und für 24 Stunden bei -20°C eingefroren. Am folgenden Tag wurde das Membransediment aufgetaut und mit eiskaltem 5 mmol/l TRIS-Acetatpuffer, 0,1 % Saponin (Gewicht/Volumen) pH 7,0 (10 ml/g ursprüngliches Frischeinwaage) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut

mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Durchführung der Untersuchung eingefroren.

Für den Rezeptorbindungstest wurden Aliquote aufgetaut, 1:10 mit 5 mmol/l TRIS-Acetatpuffer pH 7,0 verdünnt, mit 10 Kolbenhüben bei 500 Upm mit dem Potter-Homogenisator unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).

Im Bindungstest wurde als Puffer 50 mmol/l TRIS-Acetatpuffer pH 7,0 sowie als radioaktiver Ligand 1 nmol/l ($^3$H)-MDL 105.519 (Baron B.M. et al, J. Pharmacol. Exp. Ther., Vol. 279 , pp. 62-68 (1996)) eingesetzt. Der Anteil an unspezifischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (Typ Whatman GF/B, Fa. Adi Hassel, München, Deutschland) geerntet. Die auf den Glasfaser-Filtern zurückgehaltene Radioaktivität wurde nach Zugabe von Szintillator (Ready Protein, Fa. Beckamnn Coulter GmbH, Krefeld, Deutschland) im β-Counter (Packard TRI-CARB Liquid Szintillation Analyzer 2000CA, Fa. Packard Instrument, Meriden, CT 06450, USA) gemessen.

Die Affinität der erfindungsgemäßen Verbindungen zur Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und nach Umrechnung (nach der Cheng-Prussoff-Gleichung (Y. Cheng, W.H. Prusoff, 1973, Biochem. Pharmacol., Vol. 22, pp. 3099-3108)) als Ki-Wert angegeben.

b) NMDA/Glycin-induzierte Ionenströme an RNA-injizierten Xenopus Oocyten

Die Untersuchung zur Bestimmung von Funktionsänderungen des NMDA-Rezeptorkanals durch die erfindungsgemäßen Verbindungen der allgemeinen Formel Ia wurde an Oozyten des Südafrikanischen Krallenfrosches, Xenopus laevis, durchgeführt. Hierzu wurden neuronale NMDA-Rezeptorkanäle nach Injektion von RNA aus Mäusehirnen in Oozyten ausgebildet und durch Koapplikation von NMDA und Glycin ausgelöste Ionenströme gemessen.

Xenopus Oozyten der Stadien V und VI (Dumont, J.N., J. Morphol., Vol. 136, pp. 153-180 (1972)) wurden mit Gesamt-RNA aus Himgewebe adulter Mäuse mikro-injiziert (100-130 ng/Zelle) und bis zu 10 Tage in Kulturmedium (Zusammensetzung: 88.0 mmol/l NaCl, 1.0 mmol/l KCl, 1.5 mmol/l $CaCl_2$, 0.8 mmol/l $MgSO_4$, 2.4 mmol/l $NaHCO_3$, 5 mmol/l HEPES, 100 IU/ml Penicillin, 100 µg/ml Streptomycin, pH 7.4) bei 20 °C gehalten. Transmembranöse Ionenströme wurden mit Hilfe der konventionellen Zwei-Elektroden-Spannungsklemmtechnik bei einem Haltepotential von -70 mV registriert (Bloms-Funke P. et al, (1996) Neurosci. Lett. 205, pp. 115-118 (1996)). Zur Datenaufzeichnung und Steuerung der Versuchsapparatur wurden das OTC-Interface und die Software Cellworks verwendet (Firma npi, Bundesrepublik Deutschland). Die erfindungsgemäßen Verbindungen wurden einem nominal $Mg^{2+}$-freien Medium (Zusammensetzung: 89.0 mmol/l NaCl, 1.0 mmol/l KCl, 1.8 mmol/l $CaCl_2$, 2.4 mmol/l $NaHCO_3$, 5 mmol/l HEPES, pH 7.4) zugesetzt und mit Hilfe einer Konzentrationsklemme systemisch appliziert (Firma npi, Bundesrepublik Deutschland). Um Substanzeffekte zu testen, die über die Glycin B-Bindungsstelle des NMDA-Rezeptorkanals vermittelt sind, wurde die Glycin-Dosiswirkungskurve mit und ohne die jeweilige erfindungsgemäße Verbindung aufgezeichnet. Dazu wurde NMDA in einer fixen Konzentration von 100 µmol/l mit Glycin in steigenden Konzentrationen (0-100 µmol/l) kumulativ koappliziert. Im Anschluß wurde das Experiment in gleicher Weise mit einer festen Konzentration der erfindungsgemäßen Verbindung wiederholt. Die Stromamplituden wurden auf die der Kontrollantwort auf Koapplikation von NMDA (100 µmol/l) mit Glycin (10 µmol/l) normiert. Die Analyse der Daten wurde mit der Software Igor-Pro (Version 3.1, WaveMetrics, USA) durchgeführt. Alle Ergebnisse wurden als Mittelwert aus mindestens 3 Experimenten an verschiedenen Oozyten von mindestens zwei Fröschen angegeben. Die Signifikanz für ungepaarte Meßgrößen wird mit Hilfe des Mann-Whitney U-Test und für gepaarte Meßgrößen durch den Wilcoxon-Test ermittelt (Sysstat, SPSS Inc., USA). $EC_{50}$-Werte werden nach folgender Formel berechnet:

$$Y = Y_{min} + (Y_{max} - Y_{min}) / (1 + (X/EC_{50})^{-p})$$

($Y_{min}$ =minimaler Testwert; $Y_{max}$ = maximaler Testwert, Y= relative Stromamplitude, X = Konzentration der Testsubstanz, p = Slope-Faktor). Bei Rechtsverschiebung der Glycin-Dosiswirkungskurve wurde anhand einer Schild-Regression der $pA_2$-Wert der erfindungsgemäßen Verbindung graphisch ermittelt. Konzentrationsverhältnisse wurden anhand der $EC_{50}$-Werte kalkuliert, die für jede Dosiswirkungskurve unabhängig errechnet wurden.

c) Formalin-Test an der Maus

Die Untersuchungen zur Bestimmung der antinociceptiven Wirkung der erfindungsgemäßen substituierten 1,5-Dihydropyrrol-2-on-Derivate wurden im Formalin-Test an männlichen Albino-Mäusen (NMRI, 25 - 35 g, Iffa Credo, Belgien) durchgeführt.

Im Formalin-Test werden die erste (frühe) Phase (0 - 15 min nach Formalin-Injektion) und die zweite (späte) Phase (15 - 60 min nach Formalin-Injektion) unterschieden (D. Dubuisson er al, Pain, Vol. 4, pp. 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre et al, Pain, Vol. 52, pp. 259 - 285 (1993)).

Die erfindungsgemäßen Verbindungen wurden in der zweiten Phase des Formalin-Tests untersucht, um Aussagen über Substanzwirkungen im chronisch/entzündlichen Schmerz zu erhalten.
Durch eine einmalige subkutane Formalin-Injektion (20 µl, 1 %ige wäßrige Lösung) in die dorsale Seite der rechten Hinterpfote wurde bei freibeweglichen Versuchstieren eine nociceptive Reaktion induziert, die sich in deutlichem Lekken und Beißen der betroffenen Pfote äußert.
Für den Untersuchungszeitraum in der zweiten (späten) Phase des Formalin-Tests wurde das nozizeptive Verhalten durch Beobachtung der Tiere kontinuierlich erfaßt. Die Quantifizierung des Schmerzverhaltens erfolgte durch Summation der Sekunden, in denen die Tiere im Untersuchungszeitraum Lecken und Beißen der betroffenen Pfote zeigten. Nach Injektion von Substanzen, die im Formalin-Test antinozizeptiv wirksam sind, sind die beschriebenen Verhaltensweisen der Tiere reduziert, evtl. sogar aufgehoben. Entsprechend den Substanzversuchen, bei denen die Tiere Testsubstanz vor Formalin injiziert bekommen hatten, wurde den Kontrolltieren Vehikel, d.h. Lösungmittel (z.B. 0,9%ige NaCl-Lösung), vor der Formalin-applikation gespritzt. Das Verhalten der Tiere nach Substanzgabe (10 Mäuse pro Substanzdosierung) wurde mit einer Kontrollgruppe (10 Mäuse) verglichen.

Basierend auf der Quantifizierung des Schmerzverhaltens wurde die Substanzwirkung im Formalin-Test als Änderung der Kontrolle in Prozent ermittelt. Die $ED_{50}$-Berechnungen erfolgten mittels Regressionsanalyse. Abhängig von der Applikationsart der erfindungsgemäßen Verbindungen wurde der Applikationszeitpunkt vor der Formalin-Injektion gewählt (intraperitoneal: 15 min, intravenös: 5 min).

Im folgenden wird die Erfindung anhand von Beispielen erläutert, diese schränken aber den allgemeinen Erfindungsgedanken nicht ein.

**Beispiele**

Die Ausbeuten der hergestellten Verbindungen sind nicht optimiert.

Die Schmelzpunkte sind unkorrigiert.

Beispiel 1

5-Benzyliden-4-hydroxy-3-nitroso-1,5-dihydropyrrol-2-on

Eine wäßrige Lösung von Natriumnitrit (0,075 g, 1,1 mmol) wurde unter Rühren zu einer eisgekühlten Suspension von 2 mmol (0,432 g) 5-Benzyliden-4-hydroxy-1,5-dihydropyrrol-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in 5 ml Eisessig gegeben und 30 Minuten bei Raumtemperatur gerührt. Die Reaktionslösung wurde eingeengt und durch Umkristallisation aus Ethanol wurden orange Kristalle des 5-Benzyliden-4-hydroxy-3-nitroso-1,5-dihydropyrrol-2-on in einer Ausbeute von 65 % erhalten. Die Substanz zersetzt sich bei 205 °C.

Die Analyse dieser Verbindung mittels $^1H$-NMR Spektroskopie ergab die folgenden Signale:
$^1H$-NMR (d6-DMSO, $\delta$ in ppm): 14,66 (s, 1 H); 11,25 (s, 0,66 H); 11,18 (s, 0,33 H); 7,64 - 7,31 (m, 5 H); 6,42 (s, 0,33, H); 6,36 (s, 0,66 H).

Beispiel 2:

5-Benzyliden-4-methoxy-3-nitroso-1,5-dihydropyrrol-2-on

Eine Suspension von 5-Benzyliden-4-hydroxy-3-nitroso-1,5-dihydropyrrol-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in Methanol wurde mit einem Überschuß einer etherischen Diazomethan-Lösung versetzt. Nach Abklingen der heftigen Stickstoff-Entwicklung wird die Reaktionslösung im Vakuum eingeengt und der so erhaltene Rückstand aus Methanol umkristallisiert. Es wurden rote Kristalle des 5-Benzyliden-4-methoxy-3-nitroso-1,5-dihydropyrrol-2-on mit einem Schmelzpunkt von 172 °C in einer Ausbeute von 90 % erhalten.

Die Analyse dieser Verbindung mittels $^{1}$H-NMR Spektroskopie ergab die folgenden Signale:
$^{1}$H-NMR (d6-DMSO, $\delta$ in ppm): 11,16 (s, 1 H); 7,66 - 7,33 (m, 5 H); 6,42 (s, 1 H); 4,34 (s, 3 H).

Beispiel 3:

4-Benzylamino-5-benzyliden-3-nitroso-1,5-dihydropyrrol-2-on

Eine Lösung von 0,11 g (0,5 mmol) 5-Benzyliden-4-methoxy-3-nitroso-1,5-dihydropyrrol-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in Methanol wurde zwei Minuten mit 0,25 ml (0,25 mmol) Benzylamin unter Rückfluß gekocht. Man erhielt die Verbindung 4-Benzylamino-5-benzyliden-3-nitroso-1,5-dihydropyrrol-2-on nach Umkristallisieren aus Methanol als rot-violette Kristalle in einer Ausbeute von 60 %. Die Verbindung zersetzt sich bei 220 °C.

Die Analyse dieser Verbindung mittels $^{1}$H-NMR Spektroskopie ergab die folgenden Signale:
$^{1}$H-NMR (d6-DMSO, $\delta$ in ppm): 10,06 (s, 1 H); 8,09 (s, 1 H); 7,57-7,23 (m, 10 H); 6,09 (s, 1 H); 4,06 (s, 2 H).

Beispiel 4:

5-Benzyliden-3-nitroso-4-phenylamino-1,5-dihydrooyrrol-2-on

Eine Lösung von 0,11 g (0,5 mmol) 5-Benzyliden-4-methoxy-3-nitroso-1,5-dihydropyrrol-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in Methanol wurde zwei Minuten mit 0,25 ml (0,25 mmol) Anilin unter Rückfluß gekocht. Man erhielt die Verbindung 5-Benzyliden-3-nitroso-4-phenylamino-1,5-dihydropyrrol-2-on nach Umkristallisieren aus Methanol in einer Ausbeute von 30 %. Die Verbindung zersetzt sich bei 230 °C.

Die Analyse dieser Verbindung mittels $^{1}$H-NMR Spektroskopie ergab die folgenden Signale:
$^{1}$H-NMR (d6-DMSO, $\delta$ in ppm): 10,04 (s, 1 H); 7,68-7,11 (m, 10 H); 6,10 (s, 1H).

Beispiel 5:

5-Benzyliden-4-methylamino-3-nitroso-1,5-dihydropyrrol-2-on

Eine Lösung von 0,11 g (0,5 mmol) 5-Benzyliden-4-methoxy-3-nitroso-1,5-dihydropyrrol-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in Methanol wurde zwei Minuten mit 0,25 ml (0,25 mmol) Methylamin-Lösung unter Rückfluß gekocht. Man erhielt die Verbindung 5-Benzyliden-4-methylamino-3-nitroso-1,5-dihydropyrrol-2-on nach Umkristallisieren aus Methanol in einer Ausbeute von 50 %. Die Verbindung zersetzt sich bei 220 °C.

Die Analyse dieser Verbindung mittels $^{1}$H-NMR Spektroskopie ergab die folgenden Signale:
$^{1}$H-NMR (d6-DMSO, $\delta$ in ppm): 7.57-7.21 (m, 5 H); 6,09 (s, 1H); 3,30 (s, 3H).

Beispiel 6:

4-Amino-5-benzyliden-3-nitroso-1,5-dihydropyrrol-2-on

Eine Lösung von 0,11 g (0,5 mmol) 5-Benzyliden-4-methoxy-3-nitroso-1,5-dihydropyrrol-2-on (hergestellt

nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in Methanol wurde zwei Minuten mit 0,25 ml (0,25 mmol) einer wäßrigen Ammoniak-Lösung unter Rückfluß gekocht. Man erhielt die Verbindung 4-Amino-5-benzyliden-3-nitroso-1,5-dihydropyrrol-2-on nach Umkristallisieren aus Methanol in einer Ausbeute von 45 %. Die Verbindung zersetzt sich bei 260 °C.
Die Analyse dieser Verbindung mittels $^1$H-NMR Spektroskopie ergab die folgenden Signale:
$^1$H-NMR (d6-DMSO, δ in ppm): 10,06 (s, 1H); 7.57-7.23 (m, 5 H); 6.08 (s, 1H).

Beispiel 8:

5-Benzyliden-3-nitro-4-phenylamino-1,5-dihydropyrrol-2-on

Eine Suspension von 0,46 g (2 mmol) Benzyliden-4-hydroxy-3-nitro-3-pyrrolin-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in 20 ml Methanol wird mit einem Überschuß von Diazomethan in Ether versetzt. Nach Abklingen der Stickstoffentwicklung wird das Lösungsmittel abgezogen, der Rückstand in Methanol gelöst und mit einem Überschuß Anilin fünf Minuten unter Rückfluß erhitzt, wobei gelbe Kristalle ausfallen, die abgesaugt und mit Methanol gewaschen werden. Das 5-Benzyliden-3-nitro-4-phenylamino-1,5-dihydropyrrol-2-on wurde in 50 % Ausbeute erhalten und zersetzt sich bei 236 °C.
Die Analyse dieser Verbindung mittels $^1$H-NMR Spektroskopie ergab die folgenden Signale:
$^1$H-NMR (d6-DMSO, δ in ppm): 10,82 (s, 1 H); 10,08 (s, 1 H); 7.48-7.30 (m, 10 H); 6.29 (s, 1 H).

Beispiel 9:

4-Benzylamino-5-benzyliden-3-nitro-1,5-dihydropyrrol-2-on

Eine Suspension von 0,46 g (2 mmol) Benzyliden-4-hydroxy-3-nitro-3-pyrrolin-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in 20 ml Methanol wird mit einem Überschuß von Diazomethan in Ether versetzt. Nach Abklingen der Stickstoffentwicklung wird das Lösungsmittel abgezogen, der Rückstand in Methanol gelöst und mit einem Überschuß Benzylamin fünf Minuten unter Rückfluß erhitzt, wobei gelbe Kristalle ausfallen, die abgesaugt und mit Methanol gewaschen werden. Das 4-benyzlamino-5-benzyliden-3-nitro-1,5-dihydropyrrol-2-on wurde mit einem Schmelzpunkt von 218 °C in 60 % Ausbeute erhalten.
Die Analyse dieser Verbindung mittels $^1$H-NMR Spektroskopie ergab die folgenden Signale:
$^1$H-NMR (d6-DMSO, δ in ppm): 7.43-7.32 (m, 10 H); 6.69 (s, 1H); 5,11 (s, 2H).

Beispiel 10:

5-Benzyliden-4-methylamino-3-nitro-1,5-dihydropyrrol-2-on

Eine Suspension von 0,46 g (2 mmol) Benzyliden-4-hydroxy-3-nitro-3-pyrrolin-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in 20 ml Methanol wird mit einem Überschuß von Diazomethan in Ether versetzt. Nach Abklingen der Stickstoffentwicklung wird das Lösungsmittel abgezogen, der Rückstand in Methanol gelöst und mit einem Überschuß Methylamin-Lösung fünf Minuten unter Rückfluß erhitzt, wobei gelbe Kristalle ausfallen, die abgesaugt und mit Methanol gewaschen werden. Das 5-Benzyliden-4-methylamino-3-nitro-1,5-dihydropyrrol-2-on wurde mit einem Schmelzpunkt von 238 °C in 45 % Ausbeute erhalten.
Die Analyse dieser Verbindung mittels $^1$H-NMR Spektroskopie ergab die folgenden Signale:
$^1$H-NMR (d6-DMSO, δ in ppm): 8,85 (s, 1 H); 7.42-7.27 (m, 5 H); 6.32 (s, 1 H); 3,45 (s, 3 H).

Beispiel 11:

4-Amino-5-benzyliden-3-nitro-1,5-dihydropyrrol-2-on

Eine Suspension von 0,46 g (2 mmol) Benzyliden-4-hydroxy-3-nitro-3-pyrrolin-2-on (hergestellt nach H. Poschenrieder et al (Arch. Pharm. Pharm. Med. Chem. 1998, Vol. 331, pp. 389-394) und Stachel et al (J. Heterocycl. Chem. 1980, Vol. 17, pp. 1195-1199 und Liebigs Ann. Chem. 1985, pp. 1692-1696)) in 20 ml Methanol wird mit einem Überschuß von Diazomethan in Ether versetzt. Nach Abklingen der Stickstoffentwicklung wird das Lösungsmittel abgezogen, der Rückstand in Methanol gelöst und mit einem Überschuß Ammoniak-Lösung fünf Minuten unter Rückfluß erhitzt, wobei gelbe Kristalle ausfallen, die abgesaugt und mit Methanol gewaschen werden. Das 4-Amino-5-benzyliden-3-nitro-1,5-dihydropyrrol-2-on wurde mit einem Schmelzpunkt von > 260 °C in 75 % Ausbeute erhalten.

Die Analyse dieser Verbindung mittels $^1$H-NMR Spektroskopie ergab die folgenden Signale:
$^1$H-NMR (d6-DMSO, $\delta$ in ppm): 9,92 (s, 1 H); 9,49 (s, 1 H); 9,10 (s, 1 H); 7,66-7,34 (m, 5 H); 7,00 (s, 1H).

Pharmakologische Untersuchungen

b) Untersuchungen zur Rezeptorbindung

Die Untersuchungen zur Bestimmung der Affinität der erfindungsgemäßen Verbindungen gemäß den Beispielen 3 bis 6 und 8 bis 11 zur Glycin-Bindungsstelle des NMDA-Rezeptorkanals wurde wie obenstehend beschrieben durchgeführt.

Die Affinität zur Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und ist in der nachfolgenden Tabelle 1, nach Umrechnung (nach der Cheng-Prusoff-Gleichung (Y. Cheng, W.H. Prusoff, 1973, Biochem. Pharmacol., Vol. 22, pp. 3099-3108)) als Ki-Wert angegeben.

Tabelle 1

| Beispiel | Glycin-Bindungsstelle des NMDA-Rezeptorkanals Ki (µmol/l) |
|---|---|
| 3 | 68 |
| 4 | 72 |
| 5 | 60 |
| 6 | 70 |
| 8 | 9 |
| 9 | 5 |
| 10 | 3 |
| 11 | 6 |

b) Formalin-Test an der Maus

Die Untersuchungen zur Bestimmung der antinociceptiven Wirkung der erfindungsgemäßen Verbindungen wurden wie obenstehend beschrieben durchgeführt.

Die entsprechenden Ergebnisse im Formalin-Test an der Maus sind in der nachfolgenden Tabelle 2 zusammengefaßt.

Tabelle 2:

| Beispiel | % Änderung gegen Kontrolle bei 10 mg/kg |
|---|---|
| 1 | 63,9 |
| 2 | 36,3 |
| 3 | 47,6 |

**Patentansprüche**

1. Substituierte 1,5-Dihydropyrrol-2-on-Derivate der allgemeinen Formel I,

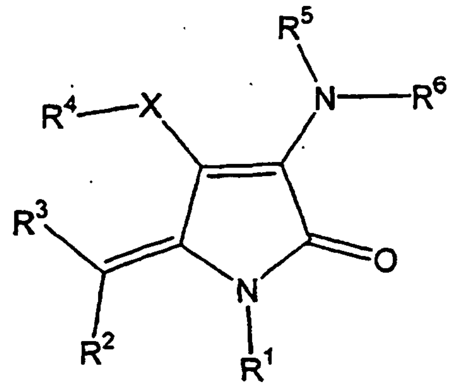

I

worin
X für O oder $NR^7$ steht,
der Rest $R^1$ für H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, einen $C_{1-10}$-Alkyl-, einen Aryl-, einen Heteroaryl- oder für einen über eine $C_{1-6}$-Alkylen-Gruppe gebundenen Aryl-Rest steht,
die Reste $R^2$, $R^3$, gleich oder verschieden, für H, F, Cl, Br, $CF_3$, $OR^{11}$, $SR^{11}$, $NR^9R^{10}$, einen $C_{1-10}$-Alkyl-, einen Aryl-, einen Heteroaryl-Rest oder für einen über eine $C_{1-6}$-Alkylen-Gruppe gebundenen Aryl-Rest stehen,
der Rest $R^4$ für H, OH, $OR^{11}$, $SR^{11}$, $COR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
die Reste $R^5$, $R^6$, gleich oder verschieden, für H, O, OH, $OR^{11}$, $SR^{11}$, $COR^8$, $CSR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest stehen oder die Reste $R^5$ und $R^6$ zusammen die Gruppe =O bedeuten,
der Rest $R^7$ für H, $OR^{11}$, $SR^{11}$, $COR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^8$ für H, $OR^{11}$, $SR^{11}$, $NR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^9$ für einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^{10}$ für einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^{11}$ für einen $C_{1-10}$-Alkyl-, einen Aryl-, einen Heteroaryl- oder für einen über eine $C_{1-6}$-Alkylen-Gruppe gebundenen Aryl-Rest steht,
in Form ihrer Racemate, Enantiomere, Diastereomere oder einer entsprechenden Base oder eines entsprechenden physiologisch verträglichen Salzes,
wobei die Verbindung
3-Acetamido-5-(ethoxymethylen)-4-hydroxy-1-methyl-3-Pyrrolin-2-on-acetat
sowie die Racemate
der 1-Methyl-3-acetamino-5-ethoxymethylentetramsäure,
der 3-Acetamino-5-ethoxymethylentetramsäure
und
der $\alpha$-Acetylamino-$\gamma$-benzylthiomethylentetramsäure
ausgenommen sind.

2. Substituierte 1,5-Dihydropyrrol-2-on-Derivate gemäß Anspruch 1, **dadurch gekennzeichnet, daß** der Rest $R^1$ für einen $C_{1-6}$-Alkylrest steht und die übrigen Reste $R^2$ bis $R^{11}$ die Bedeutung gemäß der allgemeinen Formel I haben.

3. Substituierte 1,5-Dihydropyrrol-2-on-Derivate gemäß Anspruch 1, **dadurch gekennzeichnet, daß** der Rest $R^1$ für einen über eine $C_{1-3}$-Alkylen-Gruppe gebundenen Aryl-Rest steht und die übrigen Reste $R^2$ bis $R^{11}$ die Bedeutung gemäß der allgemeinen Formel I haben.

4. Substituierte 1,5-Dihydropyrrol-2-on-Derivate gemäß einem der Ansprüche 1 bis 3, **dadurch gekennzeichnet, daß** der Rest $R^2$ und/oder $R^3$ für einen $C_{1-6}$-Alkylrest steht und die übrigen Reste $R^4$ bis $R^{11}$ sowie ggf. der Rest $R^2$ oder $R^3$ die Bedeutung gemäß der allgemeinen Formel I haben.

5. Substituierte 1,5-Dihydropyrrol-2-on-Derivate gemäß einem der Ansprüche 1 bis 3, **dadurch gekennzeichnet, daß** der Rest $R^2$ und/oder $R^3$ für einen über eine $C_{1-3}$-Alkylen-Gruppe gebundenen Aryl-Rest steht und die übrigen Reste $R^4$ bis $R^{11}$ sowie ggf. der Rest $R^2$ oder $R^3$ die Bedeutung gemäß der allgemeinen Formel I haben.

6. Substituierte 1,5-Dihydropyrrol-2-on-Derivate gemäß einem der Ansprüche 1 bis 5, **dadurch gekennzeichnet, daß** der Rest $R^4$ für einen $C_{1-6}$-AlkylRest steht und die übrigen Reste $R^5$ bis $R^{11}$ die Bedeutung gemäß der allgemeinen Formel I haben.

7. Substituierte 1,5-Dihydropyrrol-2-on-Derivate gemäß einem der Ansprüche 1 bis 5, **dadurch gekennzeichnet, daß** der Rest $R^4$ für einen über eine $C_{1-3}$-Alkylen-Gruppe gebundenen Aryl-Rest steht und die übrigen Reste $R^5$ bis $R^{11}$ die Bedeutung gemäß der allgemeinen Formel I haben.

8. Substituierte 1,5-Dihydropyrrol-2-on-Derivate gemäß einem der Ansprüche 1 bis 7, **dadurch gekennzeichnet, daß** die Reste $R^5$ und $R^6$ zusammen die Gruppe =O bedeuten und die übrigen Reste $R^7$ bis $R^{11}$ die Bedeutung gemäß der allgemeinen Formel I haben.

9. Substituierte 1,5-Dihydropyrrol-2-on-Derivate gemäß einem der Ansprüche 1 bis 7, **dadurch gekennzeichnet, daß** der Rest $R^5$ und/oder $R^6$ für einen $C_{1-6}$-Alkyl -Rest steht und die übrigen Reste $R^7$ bis $R^{11}$ sowie ggf. $R^5$ oder $R^6$ die Bedeutung gemäß der allgemeinen Formel I haben.

10. Substituierte 1,5-Dihydropyrrol-2-on-Derivate gemäß einem der Ansprüche 1 bis 7, **dadurch gekennzeichnet, daß** der Rest $R^5$ und/oder $R^6$ für einen über eine $C_{1-3}$-Alkylen-Gruppe gebundenen Aryl-Rest steht und die übrigen Reste $R^7$ bis $R^{11}$ sowie ggf. $R^5$ oder $R^6$ die Bedeutung gemäß der allgemeinen Formel I haben.

11. Substituierte 1,5-Dihydropyrrol-2-on-Derivate gemäß einem der Ansprüche 1 bis 10, **dadurch gekennzeichnet, daß** der Rest $R^7$ für einen $C_{1-6}$-Alkylrest steht und die übrigen Reste $R^8$ bis $R^{11}$ die Bedeutung gemäß der allgemeinen Formel I haben.

12. Substituierte 1,5-Dihydropyrrol-2-on-Derivate gemäß einem der Ansprüche 1 bis 10, **dadurch gekennzeichnet, daß** der Rest $R^7$ für einen über eine $C_{1-3}$-Alkylen-Gruppe gebundenen Aryl-Rest steht und die übrigen Reste $R^8$ bis $R^{11}$ die Bedeutung gemäß der allgemeinen Formel I haben.

13. Substituierte 1,5-Dihydropyrrol-2-on-Derivate gemäß einem der Ansprüche 1 bis 12, **dadurch gekennzeichnet, daß** der Rest $R^8$ für einen $C_{1-6}$-Alkyl-Rest steht und die übrigen Reste $R^9$ bis $R^{11}$ die Bedeutung gemäß der allgemeinen Formel I haben.

14. Substituierte 1,5-Dihydropyrrol-2-on-Derivate gemäß einem der Ansprüche 1 bis 12, **dadurch gekennzeichnet, daß** der Rest $R^8$ für einen über eine $C_{1-3}$-Alkylen-Gruppe gebundenen Aryl-Rest steht und die übrigen Reste $R^9$ bis $R^{11}$ die Bedeutung gemäß der allgemeinen Formel I haben.

15. Substituierte 1,5-Dihydropyrrol-2-on-Derivate gemäß einem der Ansprüche 1 bis 14, **dadurch gekennzeichnet, daß** der Rest $R^9$ für einen $C_{1-6}$-Alkyl- Rest steht und die übrigen Reste $R^{10}$ und $R^{11}$ die Bedeutung gemäß der allgemeinen Formel I haben.

16. Substituierte 1,5-Dihydropyrrol-2-on-Derivate gemäß einem der Ansprüche 1 bis 14, **dadurch gekennzeichnet, daß** der Rest $R^9$ für einen über eine $C_{1-3}$ Alkylen-Gruppe gebundenen Aryl-Rest steht und die übrigen Reste $R^{10}$ und $R^{11}$ die Bedeutung gemäß der allgemeinen Formel I haben.

17. Substituierte 1,5-Dihydropyrrol-2-on-Derivate gemäß einem der Ansprüche 1 bis 16, **dadurch gekennzeichnet, daß** der Rest $R^{10}$ für einen $C_{1-6}$-Alkyl -Rest steht und der Rest $R^{11}$ die Bedeutung gemäß der allgemeinen Formel I hat.

18. Substituierte 1,5-Dihydropyrrol-2-on-Derivate gemäß einem der Ansprüche 1 bis 16, **dadurch gekennzeichnet, daß** der Rest $R^{10}$ für einen über eine $C_{1-3}$-Alkylen-Gruppe gebundenen Aryl-Rest steht und der Rest $R^{11}$ die

Bedeutung gemäß der allgemeinen Formel I hat.

**19.** Substituierte 1,5-Dihydropyrrol-2-on-Derivate gemäß einem der Ansprüche 1 bis 18, **dadurch gekennzeichnet, daß** der Rest $R^{11}$ für einen $C_{1-6}$-Alkyl -Rest steht.

**20.** Substituierte 1,5-Dihydropyrrol-2-on-Derivate gemäß einem der Ansprüche 1 bis 18, **dadurch gekennzeichnet, daß** der Rest $R^{11}$ für einen über eine $C_{1-3}$-Alkylen-Gruppe gebundenen Aryl-Rest steht.

**21.** Substituierte 1,5-Dihydropyrrol-2-on-Derivate gemäß Anspruch 1
5-Benzyliden-4-methoxy-3-nitroso-1,5-dihydropyrrol-2-on,
4-Benzylamin-5-benzyliden-3-nitroso-1,5-dihydropyrrol-2-on,
5-Benzyliden-4-hydroxy-3-nitroso-1,5-dihydro-pyrrol-2-on,
5-Benzyliden-3-nitroso-4-phenylamino-1,5-dihydropyrrol-2-on,
5-Benzyliden-4-methylamino-3-nitroso-1,5-dihydropyrrol-2-on,
4-Amino-5-benzyliden-3-nitroso-1,5-dihydropyrrol-2-on,
5-Benzyliden-3-nitro-4-phenylamino-1,5-dihydropyrrol-2-on,
4-Benzylamino-5-benzyliden-3-nitro-1,5-dihydropyrrol-2-on,
5-Benzyliden-4-methylamino-3-nitro-1,5-dihydropyrrol-2-on,
und
4-Amino-5-benzyliden-3-nitro-1,5-dihydropyrrol-2-on.

**22.** Verfahren zur Herstellung von substituierten 1,5-Dihydropyrrol-2-on-Derivaten der allgemeinen Formel I gemäß Anspruch 1, **dadurch gekennzeichnet, daß** man Tetramsäuren der allgemeinen Formel II,

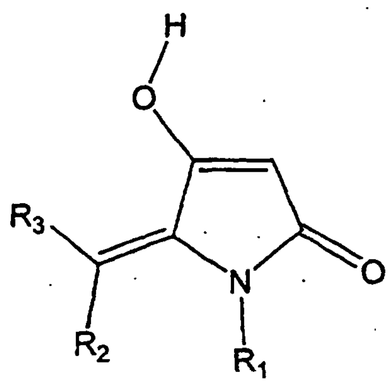

II

worin die Reste $R^1$ bis $R^3$ die Bedeutung gemäß der allgemeinen Formel I haben, bei niedriger Temperatur in Lösung, vorzugsweise in eisgekühlter saurer Lösung, besonders bevorzugt in eisgekühlter, saurer Lösung von Eisessig mit einer wäßrigen Lösung eines Alkalinitrits, vorzugsweise Natriumnitrits zu Verbindungen der allgemeinen Formel III,

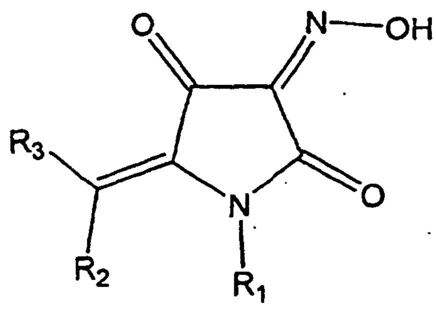

III

worin $R^1$ bis $R^3$ die Bedeutung gemäß der allgemeinen Formel I haben, umsetzt, und diese vorzugsweise durch Umkristallisation, bevorzugt aus Ethanol reinigt und isoliert,
und diese durch Alkylierung mit Alkylierungsmitteln oder durch die Umsetzung mit Säurechloriden, Säurebromiden, Chlorkohlensäureestern, Fluorkohlensäureestern, Isocyanaten und/oder Isothiocyanaten in unpolaren Lösungsmitteln und/oder polaren, aprotischen Lösungsmitteln und/oder polaren, protischen Lösungsmitteln zu Verbindungen der allgemeinen Formel IV

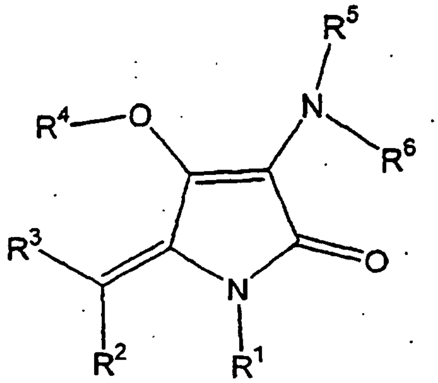

IV

IV

umsetzt, worin die Reste $R^1$ bis $R^4$ die Bedeutung gemäß der allgemeinen Formel I haben und die Reste $R^5$ und $R^6$ zusammen die Gruppe =O bedeuten, und diese nach üblichen Methoden reinigt und isoliert.

**23.** Verfahren zur Herstellung von substituierten 1,5-Dihydropyrrol-2-on-Derivaten der allgemeinen Formel I gemäß Anspruch 1, **dadurch gekennzeichnet, daß** man die Verbindungen der allgemeinen Formel IV gemäß Anspruch 22, worin die Reste $R^1$ bis $R^4$ die Bedeutung gemäß der allgemeinen Formel I haben und die Reste $R^5$ und $R^6$ zusammen die Gruppe =O bedeuten, durch Umsetzung mit Nukleophilen in polaren Lösungsmitteln zu Verbindungen der allgemeinen Formel V

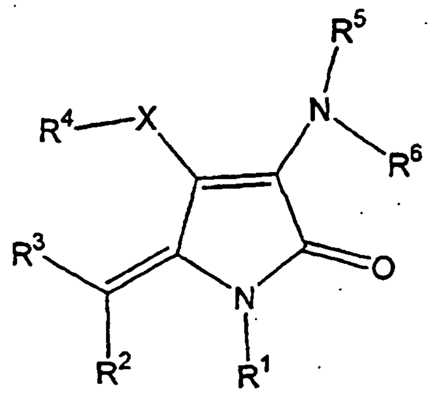

V

umsetzt, worin die Reste $R^1$ bis $R^4$ sowie X die Bedeutung gemäß der allgemeinen Formel I haben und die Reste $R^5$ und $R^6$ zusammen .die Gruppe =O bedeuten, und diese durch durch Umkristallisation, vorzugsweise aus Methanol, Ethanol und/oder Isopropanol reinigt und isoliert.

**24.** Verfahren zur Herstellung von substituierten 1,5-Dihydropyrrol-2-on-Derivaten der allgemeinen Formel I gemäß Anspruch 1, **dadurch gekennzeichnet, daß** man die Verbindungen der allgemeinen Formel V gemäß Anspruch 23 zu Verbindungen der allgemeinen Formel I, worin $R^5$ und $R^6$ jeweils H bedeuten und $R^1$ bis $R^4$ sowie X die Bedeutung gemäß der allgemeinen Formel I haben, reduziert, und diese nach üblichen Methoden reinigt und isoliert.

**25.** Verfahren gemäß Anspruch 22, **dadurch gekennzeichnet, daß** man als Alkylierungsmittel Diazoalkane, Dialkylsulfate, Alkylhalogenide, oder ein Gemisch zwei dieser Verbindungen, vorzugsweise Diazoalkane in etherischer Lösung einsetzt.

**26.** Verfahren gemäß Anspruch 22, **dadurch gekennzeichnet, daß** als unpolare Lösungsmittel offenkettige und/oder cyclische Ether, Kohlenwasserstoffe und/oder halogenhaltigen Kohlenwasserstoffe, als polare, aprotische Lösungsmittel Dimethylformamid und/oder N-Methylpyrrolidon und/oder als polares, protisches Lösungsmittel Dimethylsulfoxid eingesetzt werden.

**27.** Verfahren gemäß Anspruch 23, **dadurch gekennzeichnet, daß** als Nukleophile primäre oder sekundäre Amine oder aliphatische, aromatische oder heteroaromatische Alkoholate oder Phenolate eingesetzt werden.

**28.** Verfahren gemäß Anspruch 23, **dadurch gekennzeichnet, daß** als polares Lösungsmittel Methanol, Ethanol und/ oder Isopropanol eingesetzt wird.

**29.** Verfahren gemäß Anspruch 24, **dadurch gekennzeichnet, daß** man zur Reduktion Zn/Eisessig, Lithiumaluminiumhydrid, $BH_3$, $Na[BH_3CN]/TiCl_3$, Natriumalkoxide oder Wasserstoff in Gegenwart von Übergangsmetallkatalysatoren einsetzt.

**30.** Arzneimittel enthaltend als pharmazeutischen Wirkstoff wenigstens ein substituiertes 1,5-Dihydropyrrol-2-on-Derivat der allgemeinen Formel Ia

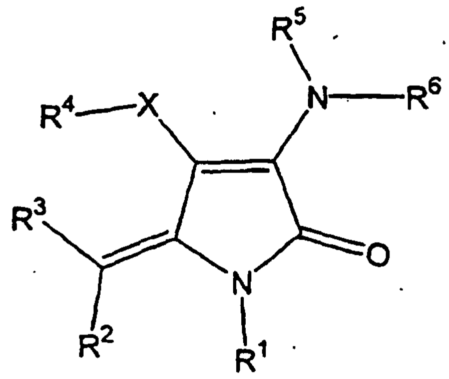

Ia

worin
X für O, S oder $NR^7$ steht,
der Rest $R^1$ für H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, einen $C_{1-10}$-Alkyl-, einen Aryl-, einen Heteroaryl- oder für einen über eine $C_{1-6}$-Alkylen-Gruppe gebundenen Aryl-Rest steht,
die Reste $R^2$, $R^3$, gleich oder verschieden, für H, F, Cl, Br, $CF_3$, $OR^{11}$, $SR^{11}$, $NR^9R^{10}$, einen $C_{1-10}$-Alkyl-, einen Aryl-, einen Heteroaryl-Rest oder für einen über eine $C_{1-6}$-Alkylen-Gruppe gebundenen Aryl-Rest stehen,
der Rest $R^4$ für H, OH, $OR^{11}$, $SR^{11}$, $COR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
die Reste $R^5$, $R^6$, gleich oder verschieden, für H, O, OH, $OR^{11}$, $SR^{11}$, $COR^8$, $CSR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest stehen oder die Reste $R^5$ und $R^6$ zusammen die Gruppe =O bedeuten,
der Rest $R^7$ für H, $OR^{11}$, $SR^{11}$, $COR^8$, $COOR^8$, $COCOR^8$, $CONR^9R^{10}$, $CSNR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^8$ für H, $OR^{11}$, $SR^{11}$, $NR^9R^{10}$, einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^9$ für einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^{10}$ für einen $C_{1-10}$-Alkyl-, einen Aryl-, einen Heteroaryl- oder für einen über eine $C_{1-6}$-Alkylen-Gruppe gebundenen Aryl-Rest steht,
der Rest $R^{11}$ für einen $C_{1-10}$-Alkyl-, einen Aryl-, einen Heteroaryl- oder für einen über eine $C_{1-6}$-Alkylen-Gruppe gebundenen Aryl-Rest steht,
in Form seines Racemates, seiner Enantiomere, seiner Diastereomere oder einer entsprechenden Base oder eines entsprechenden physiologisch verträglichen Salzes sowie gegebenfalls weitere Wirkstoffe und/oder Hilfsstoffe.

**31.** Arzneimittel nach Anspruch 30 zur Behandlung/Bekämpfung bei/von Schmerzen, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen und/oder Epilepsie.

**32.** Arzneimittel nach Anspruch 30 zur Behandlung/Prophylaxe von/bei Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Hirnödemen, Hypoxie, Anoxie, AIDS-Demens, Encephalomyelitis, Tourette-Syndrom, perinataler Asphyxie, Tinnitus aurium und/oder zur Anxiolyse.

**33.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Bekämpfung von Schmerzen.

**34.** Verwendung wenigstens eines substituierten 1,5-Dihydropyrrol-2-on-Derivates der allgemeinen Formel Ia gemäß

Anspruch 30 und/oder einer entsprechenden Base und/oder eines entsprechenden physiologisch verträglichen Salzes zur Herstellung eines Arzneimittels zur Behandlung von inflammatorischen Reaktionen.

**35.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von allergischen Reaktionen.

**36.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Depressionen.

**37.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Drogen- und/oder Alkoholmißbrauch.

**38.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Gastritis.

**39.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Diarrhoe.

**40.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Harninkontinenz.

**41.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von cardiovaskulären Erkrankungen.

**42.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Atemwegserkrankungen.

**43.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Husten.

**44.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von seelischen Erkrankungen.

**45.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Epilepsie.

**46.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Schizophrenie.

**47.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Morbus Alzheimer.

**48.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Morbus Huntington.

**49.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Morbus Parkinson.

**50.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von cerebralen Ischämien.

**51.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von cerebralen Infarkten.

**52.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Psychosen bedingt durch erhöhten Aminosäurespiegel.

**53.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Prophylaxe bei Schlaganfällen.

**54.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Hirnödemen.

**55.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Hypoxie.

**56.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Anoxie.

**57.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von AIDS-Demens.

**58.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von Enephalomyelitis.

**59.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung bei Tourette-Syndrom.

**60.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung von perinataler Asphyxie.

**61.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur anxiolytischen Behandlung.

**62.** Verwendung wenigstens eines substituierten 1,5-Dihydropyrrol-2-on-Derivates der allgemeinen Formel Ia gemäß Anspruch 30 und/oder einer entsprechenden Base und/oder eines entsprechenden physiologisch verträglichen Salzes zur Herstellung eines Arzneimittels zur Behandlung bei Tinnitus aurium.

## Claims

1. Substituted 1,5-dihydropyrrol-2-one derivatives of the general formula I

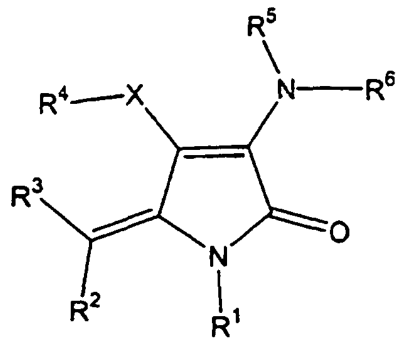

I

wherein
X represents O or $NR^7$,
the radical $R^1$ represents H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, a $C_{1-10}$-alkyl, an aryl or a heteroaryl radical or represents an aryl radical bonded via a $C_{1-6}$-alkylene group
the radicals $R^2$, $R^3$, which are identical or different, represent H, F, Cl, Br, $CF_3$, $OR^{11}$, $SR^{11}$, $NR^9R^{10}$, a $C_{1-10}$-alkyl, an aryl or a heteroaryl radical or represent an aryl radical bonded via a $C_{1-6}$-alkylene group,
the radical $R^4$ represents H, OH, $OR^{11}$, $SR^{11}$, $COR^8$, $COOR^8$, $COCOR^8$, $CONR^9R^{10}$, $CSNR^9R^{10}$, a $C_{1-10}$-alkyl, an aryl or a heteroaryl radical or represents an aryl radical bonded via a $C_{1-6}$-alkylene group,
the radicals $R^5$, $R^6$, which are identical or different, represent H, O, OH, $OR^{11}$, $SR^{11}$, $COR^8$, $CSR^8$, $COOR^8$, $COCOR^8$, $CONR^9R^{10}$, $CSNR^9R^{10}$, a $C_{1-10}$-alkyl, an aryl or a heteroaryl radical or represent an aryl radical bonded via a $C_{1-6}$-alkylene group or the radicals $R^5$ and $R^6$ together denote the group =O,
the radical $R^7$ represents H, $OR^{11}$, $SR^{11}$, $COR^8$, $COOR^8$, $COCOR^8$, $CONR^9R^{10}$, $CSNR^9R^{10}$, a $C_{1-10}$-alkyl, an aryl or a heteroaryl radical or represents an aryl radical bonded via a $C_{1-6}$-alkylene group,
the radical $R^8$ represents H, $OR^{11}$, $SR^{11}$, $NR^9R^{10}$, a $C_{1-10}$-alkyl, an aryl or a heteroaryl radical or represents an aryl radical bonded via a $C_{1-6}$-alkylene group,
the radical $R^9$ represents a $C_{1-10}$-alkyl, an aryl or a heteroaryl radical or represents an aryl radical bonded via a $C_{1-6}$-alkylene group,
the radical $R^{10}$ represents a $C_{1-10}$-alkyl, an aryl or a heteroaryl radical or represents an aryl radical bonded via a $C_{1-6}$-alkylene group,
the radical $R^{11}$ represents a $C_{1-10}$-alkyl, an aryl or a heteroaryl radical or represents an aryl radical bonded via a $C_{1-6}$-alkylene group,
in the form of their racemates, enantiomers, diastereomers or a corresponding base or a corresponding physiologically tolerated salt,
excluding the compound
3-acetamido-5-(ethoxymethylene)-4-hydroxy-1-methyl-3-Pyrrolin-2-one acetate
and the racemates
of 1-methyl-3-acetamino-5-ethoxymethylenetetramic acid,
of 3-acetamino-5-ethoxymethylenetetramic acid
and
of α-acetylamino-γ-benzylthiomethylenetetramic acid.

2. Substituted 1,5-dihydropyrrol-2-one derivatives according to claim 1, **characterized in that** the radical $R^1$ represents a $C_{1-6}$-alkyl radical and the other radicals $R^2$ to $R^{11}$ have the meaning according to the general formula I.

3. Substituted 1,5-dihydropyrrol-2-one derivatives according to claim 1, **characterized in that** the radical $R^1$ represents an aryl radical bonded via a $C_{1-3}$-alkylene group and the other radicals $R^2$ to $R^{11}$ have the meaning according to the general formula I.

4. Substituted 1,5-dihydropyrrol-2-one derivatives according to one of claims 1 to 3, **characterized in that** the radical $R^2$ and/or $R^3$ represents a $C_{1-6}$-alkyl radical and the other radicals $R^4$ to $R^{11}$ and where appropriate the radical $R^2$ or $R^3$ have the meaning according to the general formula I.

5. Substituted 1,5-dihydropyrrol-2-one derivatives according to one of claims 1 to 3, **characterized in that** the radical $R^2$ and/or $R^3$ represents an aryl radical bonded via a $C_{1-3}$-alkylene group and the other radicals $R^4$ to $R^{11}$ and where appropriate the radical $R^2$ or $R^3$ have the meaning according to the general formula I.

6. Substituted 1,5-dihydropyrrol-2-one derivatives according to one of claims 1 to 5, **characterized in that** the radical $R^4$ represents a $C_{1-6}$-alkyl radical and the other radicals $R^5$ to $R^{11}$ have the meaning according to the general formula I.

7. Substituted 1,5-dihydropyrrol-2-one derivatives according to one of claims 1 to 5, **characterized in that** the radical $R^4$ represents an aryl radical bonded via a $C_{1-3}$-alkylene group and the other radicals $R^5$ to $R^{11}$ have the meaning according to the general formula I.

8. Substituted 1,5-dihydropyrrol-2-one derivatives according to one of claims 1 to 7, **characterized in that** the radicals $R^5$ and $R^6$ together denote the group =O and the other radicals $R^7$ to $R^{11}$ have the meaning according to the general formula I.

9. Substituted 1,5-dihydropyrrol-2-one derivatives according to one of claims 1 to 7, **characterized in that** the radical $R^5$ and/or $R^6$ represents a $C_{1-6}$-alkyl radical and the other radicals $R^7$ to $R^{11}$ and where appropriate $R^5$ or $R^6$ have the meaning according to the general formula I.

10. Substituted 1,5-dihydropyrrol-2-one derivatives according to one of claims 1 to 7, **characterized in that** the radical $R^5$ and/or $R^6$ represents an aryl radical bonded via a $C_{1-3}$-alkylene group and the other radicals $R^7$ to $R^{11}$ and where appropriate $R^5$ or $R^6$ have the meaning according to the general formula I.

11. Substituted 1,5-dihydropyrrol-2-one derivatives according to one of claims 1 to 10, **characterized in that** the radical $R^7$ represents a $C_{1-6}$-alkyl radical and the other radicals $R^8$ to $R^{11}$ have the meaning according to the general formula I.

12. Substituted 1,5-dihydropyrrol-2-one derivatives according to one of claims 1 to 10, **characterized in that** the radical $R^7$ represents an aryl radical bonded via a $C_{1-3}$-alkylene group and the other radicals $R^8$ to $R^{11}$ have the meaning according to the general formula I.

13. Substituted 1,5-dihydropyrrol-2-one derivatives according to one of claims 1 to 12, **characterized in that** the radical $R^8$ represents a $C_{1-6}$-alkyl radical and the other radicals $R^9$ to $R^{11}$ have the meaning according to the general formula I.

14. Substituted 1,5-dihydropyrrol-2-one derivatives according to one of claims 1 to 12, **characterized in that** the radical $R^8$ represents an aryl radical bonded via a $C_{1-3}$-alkylene group and the other radicals $R^9$ to $R^{11}$ have the meaning according to the general formula I.

15. Substituted 1,5-dihydropyrrol-2-one derivatives according to one of claims 1 to 14, **characterized in that** the radical $R^9$ represents a $C_{1-6}$-alkyl radical and the other radicals $R^{10}$ and $R^{11}$ have the meaning according to the general formula I.

16. Substituted 1,5-dihydropyrrol-2-one derivatives according to one of claims 1 to 14, **characterized in that** the radical $R^9$ represents an aryl radical bonded via a $C_{1-3}$-alkylene group and the other radicals $R^{10}$ and $R^{11}$ have the meaning according to the general formula I.

17. Substituted 1,5-dihydropyrrol-2-one derivatives according to one of claims 1 to 16, **characterized in that** the radical $R^{10}$ represents a $C_{1-6}$-alkyl radical and $R^{11}$ has the meaning according to the general formula I.

18. Substituted 1,5-dihydropyrrol-2-one derivatives according to one of claims 1 to 16, **characterized in that** the radical $R^{10}$ represents an aryl radical bonded via a $C_{1-3}$-alkylene group and $R^{11}$ has the meaning according to the general formula I.

**19.** Substituted 1,5-dihydropyrrol-2-one derivatives according to one of claims 1 to 18, **characterized in that** the radical $R^{11}$ represents a $C_{1-6}$-alkyl radical.

**20.** Substituted 1,5-dihydropyrrol-2-one derivatives according to one of claims 1 to 18, **characterized in that** the radical $R^{11}$ represents an aryl radical bonded via a $C_{1-3}$-alkylene group.

**21.** Substituted 1,5-dihydropyrrol-2-one derivatives according to claim 1
5-benzylidene-4-methoxy-3-nitroso-1,5-dihydropyrrol-2-one
4-benzylamine-5-benzylidene-3-nitroso-1,5-dihydropyrrol-2-one
5-benzylidene-4-hydroxy-3-nitroso-1,5-dihydro-pyrrol-2-one
5-benzylidene-3-nitroso-4-phenylamino-1,5-dihydropyrrol-2-one,
5-benzylidene-4-methylamino-3-nitroso-1,5-dihydropyrrol-2-one,
4-amino-5-benzylidene-3-nitroso-1,5-dihydropyrrol-2-one,
5-benzylidene-3-nitro-4-phenylamino-1,5-dihydropyrrol-2-one,
4-benzylamino-5-benzylidene-3-nitro-1,5-dihydropyrrol-2-one,
5-benzylidene-4-methylamino-3-nitro-1,5-dihydropyrrol-2-one
and
4-amino-5-benzylidene-3-nitro-1,5-dihydropyrrol-2-one.

**22.** Process for the preparation of substituted 1,5-dihydropyrrol-2-one derivatives of the general formula I according to claim 1, **characterized in that** tetram acids of the general formula II

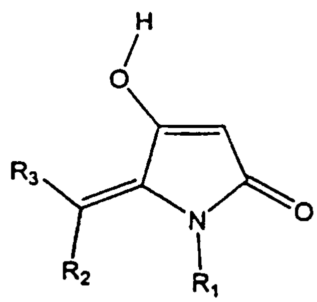

II

wherein the radicals $R^1$ to $R^3$ have the meaning according to the general formula I, are reacted with an aqueous solution of an alkali metal nitrite, preferably sodium nitrite, at a low temperature in solution, preferably in an ice-cooled acid solution, particularly preferably in an ice-cooled acid solution of glacial acetic acid, to give compounds of the general formula III

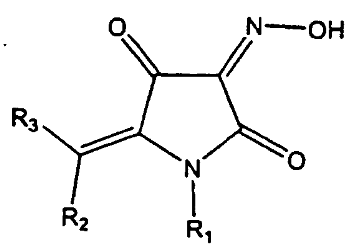

III

wherein $R^1$ to $R^3$ have the meaning according to the general formula I, and these are purified by recrystallization, preferably from ethanol, and isolated,

and these are converted by alkylation with alkylating agents or by reaction with acid chlorides, acid bromides, chlorocarbonic acid esters, fluorocarbonic acid esters, isocyanates and/or isothiocyanates in non-polar solvents and/or polar, aprotic solvents, and/or polar, protic solvents into compounds of the general formula IV

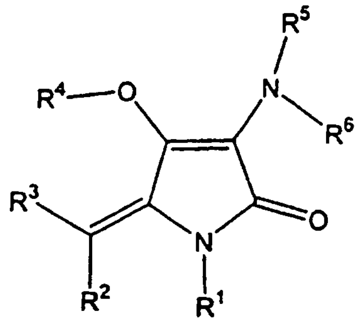

IV

IV

wherein the radicals $R^1$ to $R^4$ have the meaning according to the general formula I and the radicals $R^5$ and $R^6$ together denote the group =O, and these are purified and isolated by conventional methods.

**23.** Process for the preparation of substituted 1,5-dihydropyrrol-2-one derivatives of the general formula I according to claim 1, **characterized in that** the compounds of the general formula IV according to claim 22, wherein the radicals $R^1$ to $R^4$ have the meaning according to the general formula I and the radicals $R^5$ and $R^6$ together deonote the group =O, are converted by reaction with nucleophiles in polar solvents into compounds of the general formula V

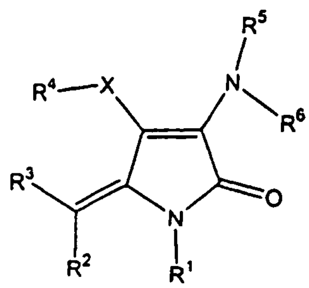

V

wherein the radicals $R^1$ to $R^4$ and X have the meaning according to the general formula I and the radicals $R^5$ and $R^6$ together denote the group =O, and these are purified by by recrystallization, preferably from methanol, ethanol and/or isopropanol, and isolated.

**24.** Process for the preparation of substituted 1,5-dihydropyrrol-2-one derivatives of the general formula I according to claim 1, **characterized in that** the compounds of the general formula V according to claim 23 are reduced to compounds of the general formula I wherein $R^5$ and $R^6$ each denote H and $R^1$ to $R^4$ and X have the meaning according to the general formula I, and these are purified and isolated by conventional methods.

**25.** Process according to claim 22, **characterized in that** diazoalkanes, dialkyl sulfates, alkyl halides, or a mixture of two of these compounds, preferably diazoalkanes in ethereal solution, are employed as alkylating agents.

**26.** Process according to claim 22, **characterized in that** open-chain and/or cyclic ethers, hydrocarbons and/or halogen-containing hydrocarbons are employed as non-polar solvents, dimethylformamide and/or N-methylpyrrolidone are employed as polar, aprotic solvents, and dimethylsulfoxide is employed as a polar, protic solvent.

**27.** Process according to claim 23, **characterized in that** primary or secondary amines or aliphatic, aromatic or heteroaromatic alcoholates or phenolates are employed as nucleophiles.

**28.** Process according to claim 23, **characterized in that** methanol, ethanol and/or isopropanol is employed as the polar solvent.

**29.** Process according to claim 24, **characterized in that** Zn/glacial acetic acid, lithium aluminium hydride, $BH_3$, Na $[BH_3CN]/TiCl_3$, sodium alkoxides or hydrogen in the presence of transition metal catalysts is employed for the reduction.

**30.** Medicaments comprising, as the pharmaceutical active compound, at least one substituted 1,5-dihydropyrrol-2-one derivative of the general formula Ia

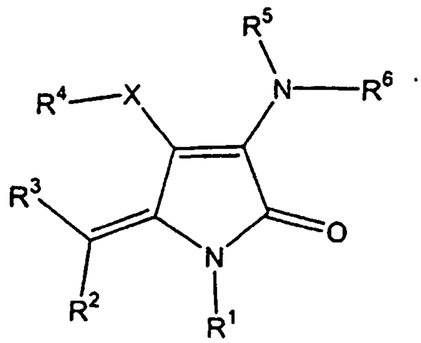

**Ia**

wherein
X represents O or $NR^7$,
the radical $R^1$ represents H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, a $C_{1-10}$-alkyl, an aryl or a heteroaryl radical or represents an aryl radical bonded via a $C_{1-6}$-alkylene group,
the radicals $R^2$, $R^3$, which are identical or different, represent H, F, Cl, Br, $CF_3$, $OR^{11}$, $SR^{11}$, $NR^9R^{10}$, a $C_{1-10}$-alkyl, an aryl or a heteroaryl radical or represent an aryl radical bonded via a $C_{1-6}$-alkylene group,
the radical $R^4$ represents H, OH, $OR^{11}$, $SR^{11}$, $COR^8$, $COOR^8$, $COCOR^8$, $CONR^9R^{10}$, $CSNR^9R^{10}$, a $C_{1-10}$-alkyl, an aryl or a heteroaryl radical or represents an aryl radical bonded via a $C_{1-6}$-alkylene group,
the radicals $R^5$, $R^6$, which are identical or different, represent H, O, OH, $OR^{11}$, $SR^{11}$, $COR^8$, $CSR^8$, $COOR^8$, $COCOR^8$, $CONR^9R^{10}$, $CSNR^9R^{10}$, a $C_{1-10}$-alkyl, an aryl or a heteroaryl radical or represent an aryl radical bonded via a $C_{1-6}$-alkylene group or the radicals $R^5$ and $R^6$ together denote the group =O,
the radical $R^7$ represents H, $OR^{11}$, $SR^{11}$, $COR^8$, $COOR^8$, $COCOR^8$, $CONR^9R^{10}$, $CSNR^9R^{10}$, a $C_{1-10}$-alkyl, an aryl or a heteroaryl radical or represents an aryl radical bonded via a $C_{1-6}$-alkylene group,
the radical $R^8$ represents H, $OR^{11}$, $SR^{11}$, $NR^9R^{10}$, a $C_{1-10}$-alkyl, an aryl or a heteroaryl radical or represents an aryl radical bonded via a $C_{1-6}$-alkylene group,
the radical $R^9$ represents a $C_{1-10}$-alkyl, an aryl or a heteroaryl radical or represents an aryl radical bonded via a $C_{1-6}$-alkylene group,
the radical $R^{10}$ represents a $C_{1-10}$-alkyl, an aryl or a heteroaryl radical or represents an aryl radical bonded via a $C_{1-6}$-alkylene group,
the radical $R^{11}$ represents a $C_{1-10}$-alkyl, an aryl or a heteroaryl radical or represents an aryl radical bonded via a $C_{1-6}$-alkylene group,
in the form of its racemate, its enantiomers, its diastereomers or a corresponding base or a corresponding physiologically tolerated salt, and optionally further active compounds and/or auxiliaries.

**31.** Medicaments according to claim 30 for treatment/control for/of pain, inflammatory and allergic reactions, depressions, drug and/or alcohol abuse, gastritis, diarrhoea, urinary incontinence, cardiovascular diseases, respiratory

tract diseases, coughing, mental illnesses and/or epilepsy.

**32.** Medicaments according to claim 30 for treatment/prophylaxis of/for schizophrenia, Alzheimer's diseases, Huntington's disease, Parkinson's disease, cerebral ischaemias, cerebral infarctions, psychoses caused by increased amino acid levels, apoplexies, cerebral oedemas, hypoxia, anoxia, AIDS dementia, encephalomyelitis, Tourette's syndrome, perinatal asphyxia, tinnitus aurium and/or for anxiolysis.

**33.** Use of at least one substituted 1,5-dihydropyrrol-2-one derivative of the general formula Ia according to claim 30 and/or a corresponding base and/or a corresponding physiologically tolerated salt for the preparation of a medicament for controlling pain.

**34.** Use of at least one substituted 1,5-dihydropyrrol-2-one derivative of the general formula Ia according to claim 30 and/or a corresponding base and/or a corresponding physiologically tolerated salt for the preparation of a medicament for treatment of inflammatory reactions.

**35.** Use of at least one substituted 1,5-dihydropyrrol-2-one derivative of the general formula Ia according to claim 30 and/or a corresponding base and/or a corresponding physiologically tolerated salt for the preparation of a medicament for treatment of allergic reactions.

**36.** Use of at least one substituted 1,5-dihydropyrrol-2-one derivative of the general formula Ia according to claim 30 and/or a corresponding base and/or a corresponding physiologically tolerated salt for the preparation of a medicament for treatment of depressions.

**37.** Use of at least one substituted 1,5-dihydropyrrol-2-one derivative of the general formula Ia according to claim 30 and/or a corresponding base and/or a corresponding physiologically tolerated salt for the preparation of a medicament for treatment of drug and/or alcohol abuse.

**38.** Use of at least one substituted 1,5-dihydropyrrol-2-one derivative of the general formula Ia according to claim 30 and/or a corresponding base and/or a corresponding physiologically tolerated salt for the preparation of a medicament for treatment of gastritis.

**39.** Use of at least one substituted 1,5-dihydropyrrol-2-one derivative of the general formula Ia according to claim 30 and/or a corresponding base and/or a corresponding physiologically tolerated salt for the preparation of a medicament for treatment of diarrhoea.

**40.** Use of at least one substituted 1,5-dihydropyrrol-2-one derivative of the general formula Ia according to claim 30 and/or a corresponding base and/or a corresponding physiologically tolerated salt for the preparation of a medicament for treatment of urinary incontinence.

**41.** Use of at least one substituted 1,5-dihydropyrrol-2-one derivative of the general formula Ia according to claim 30 and/or a corresponding base and/or a corresponding physiologically tolerated salt for the preparation of a medicament for treatment of cardiovascular diseases.

**42.** Use of at least one substituted 1,5-dihydropyrrol-2-one derivative of the general formula Ia according to claim 30 and/or a corresponding base and/or a corresponding physiologically tolerated salt for the preparation of a medicament for treatment of respiratory tract diseases.

**43.** Use of at least one substituted 1,5-dihydropyrrol-2-one derivative of the general formula Ia according to claim 30 and/or a corresponding base and/or a corresponding physiologically tolerated salt for the preparation of a medicament for treatment of coughing.

**44.** Use of at least one substituted 1,5-dihydropyrrol-2-one derivative of the general formula Ia according to claim 30 and/or a corresponding base and/or a corresponding physiologically tolerated salt for the preparation of a medicament for treatment of mental illnesses.

**45.** Use of at least one substituted 1,5-dihydropyrrol-2-one derivative of the general formula Ia according to claim 30 and/or a corresponding base and/or a corresponding physiologically tolerated salt for the preparation of a medicament for treatment of epilepsy.

**46.** Use of at least one substituted 1,5-dihydropyrrol-2-one derivative of the general formula Ia according to claim 30 and/or a corresponding base and/or a corresponding physiologically tolerated salt for the preparation of a medicament for treatment of schizophrenia.

**47.** Use of at least one substituted 1,5-dihydropyrrol-2-one derivative of the general formula Ia according to claim 30 and/or a corresponding base and/or a corresponding physiologically tolerated salt for the preparation of a medicament for treatment of Alzheimer's disease.

**48.** Use of at least one substituted 1,5-dihydropyrrol-2-one derivative of the general formula Ia according to claim 30 and/or a corresponding base and/or a corresponding physiologically tolerated salt for the preparation of a medicament for treatment of Huntington's disease.

**49.** Use of at least one substituted 1,5-dihydropyrrol-2-one derivative of the general formula Ia according to claim 30 and/or a corresponding base and/or a corresponding physiologically tolerated salt for the preparation of a medicament for treatment of Parkinson's disease.

**50.** Use of at least one substituted 1,5-dihydropyrrol-2-one derivative of the general formula Ia according to claim 30 and/or a corresponding base and/or a corresponding physiologically tolerated salt for the preparation of a medicament for treatment of cerebral ischaemias.

**51.** Use of at least one substituted 1,5-dihydropyrrol-2-one derivative of the general formula Ia according to claim 30 and/or a corresponding base and/or a corresponding physiologically tolerated salt for the preparation of a medicament for treatment of cerebral infarctions.

**52.** Use of at least one substituted 1,5-dihydropyrrol-2-one derivative of the general formula Ia according to claim 30 and/or a corresponding base and/or a corresponding physiologically tolerated salt for the preparation of a medicament for treatment of psychoses caused by increased amino acid levels.

**53.** Use of at least one substituted 1,5-dihydropyrrol-2-one derivative of the general formula Ia according to claim 30 and/or a corresponding base and/or a corresponding physiologically tolerated salt for the preparation of a medicament for prophylaxis of apoplexies.

**54.** Use of at least one substituted 1,5-dihydropyrrol-2-one derivative of the general formula Ia according to claim 30 and/or a corresponding base and/or a corresponding physiologically tolerated salt for the preparation of a medicament for treatment of cerebral oedemas.

**55.** Use of at least one substituted 1,5-dihydropyrrol-2-one derivative of the general formula Ia according to claim 30 and/or a corresponding base and/or a corresponding physiologically tolerated salt for the preparation of a medicament for treatment of hypoxia.

**56.** Use of at least one substituted 1,5-dihydropyrrol-2-one derivative of the general formula Ia according to claim 30 and/or a corresponding base and/or a corresponding physiologically tolerated salt for the preparation of a medicament for treatment of anoxia.

**57.** Use of at least one substituted 1,5-dihydropyrrol-2-one derivative of the general formula Ia according to claim 30 and/or a corresponding base and/or a corresponding physiologically tolerated salt for the preparation of a medicament for treatment of AIDS dementia.

**58.** Use of at least one substituted 1,5-dihydropyrrol-2-one derivative of the general formula Ia according to claim 30 and/or a corresponding base and/or a corresponding physiologically tolerated salt for the preparation of a medicament for treatment of encephalomyelitis.

**59.** Use of at least one substituted 1,5-dihydropyrrol-2-one derivative of the general formula Ia according to claim 30 and/or a corresponding base and/or a corresponding physiologically tolerated salt for the preparation of a medicament for treatment in cases of Tourette's syndrome.

**60.** Use of at least one substituted 1,5-dihydropyrrol-2-one derivative of the general formula Ia according to claim 30 and/or a corresponding base and/or a corresponding physiologically tolerated salt for the preparation of a medicament for treatment of perinatal asphyxia.

**61.** Use of at least one substituted 1,5-dihydropyrrol-2-one derivative of the general formula Ia according to claim 30 and/or a corresponding base and/or a corresponding physiologically tolerated salt for the preparation of a medicament for anxiolytic treatment.

**62.** Use of at least one substituted 1,5-dihydropyrrol-2-one derivative of the general formula Ia according to claim 30 and/or a corresponding base and/or a corresponding physiologically tolerated salt for the preparation of a medicament for treatment in cases of tinnitus aurium.

**Revendications**

**1.** Dérivés substitués de 5-dihydropyrrole-2-one de formule générale I,

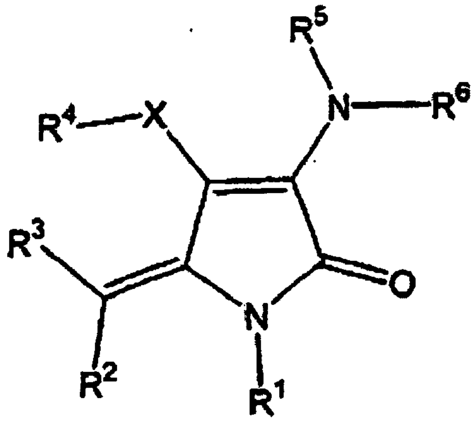

I

dans laquelle

X représente O ou $NR^7$,

le reste $R^1$ représente H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, un reste alkyle en $C_1$ à $C_{10}$, un reste aryle, un reste hétéroaryle, ou un reste aryle lié par l'intermédiaire d'un groupe alkylène en $C_1$ à $C_6$,

les restes $R^2$, $R^3$ identiques ou différents, représentent H, F, Cl, Br, $CF_3$, $OR^{11}$, $SR^{11}$, $NR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^4$ représente H, OH, $OR^{11}$, $SR^{11}$, $COR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

les restes $R^5$, $R^6$, identiques ou différents, représentent H, O, OH, $OR^{11}$, $SR^{11}$, $COR^8$, $CSR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$, ou bien les restes $R^5$ et $R^6$ représentent ensemble le groupe =O,

le reste $R^7$ représente H, $OR^{11}$, $SR^{11}$, $COR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^8$ représente H, $OR^{11}$, $SR^{11}$, $NR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^9$ représente un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^{10}$ représente un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^{11}$ représente un reste alkyle en $C_1$ à $C_{10}$, un reste aryle, un reste hétéroaryle ou un reste aryle lié par l'intermédiaire d'un groupe alkylène en $C_1$ à $C_6$,

sous forme de leurs racémates, de leurs énantiomères, de leurs diastéréo-isomères ou d'une base correspondante ou d'un sel correspondant acceptable du point de vue physiologique,

le composé

acétate de 3-acétamido-5-(éthoxyméthylène)-4-hydroxy-1-méthyl-3-pyrroline-2-one,

ainsi que les racémates

de l'acide 1-méthyl-3-acétamino-5-éthoxyméthylènetétramique,

de l'acide 3-acétamino-5-éthoxyméthylène-tétramique

et

de l'acide α-acétylamino-γ-benzylthiométhylène-tétramique,

étant exclus.

2. Dérivés substitués de 1,5-dihydropyrrole-2-one suivant la revendication 1, **caractérisés en ce que** le reste $R^1$ représente un reste alkyle en $C_1$ à $C_6$ et les autres restes $R^2$ à $R^{11}$ ont la définition indiquée conformément à la formule générale I.

3. Dérivés substitués de 1,5-dihydropyrrole-2-one suivant la revendication 1, **caractérisés en ce que** le reste $R^1$ représente un reste aryle lié par l'intermédiaire d'un groupe alkylène en $C_1$ à $C_3$ et les autres restes $R^2$ à $R^{11}$ ont la définition indiquée conformément à la formule générale I.

4. Dérivés substitués de 1,5-dihydropyrrole-2-one suivant l'une des revendications 1 à 3, **caractérisés en ce que** le reste $R^2$ et/ou le reste $R^3$ représentent un reste alkyle en $C_1$ à $C_6$ et les autres restes $R^4$ à $R^{11}$ ainsi que, le cas échéant, le reste $R^2$ ou $R^3$ ont la définition indiquée conformément à la formule générale I.

5. Dérivés substitués de 1,5-dihydropyrrole-2-one suivant l'une des revendications 1 à 3, **caractérisés en ce que** le reste $R^2$ et/ou le reste $R^3$ représentent un reste aryle lié par l'intermédiaire d'un groupe alkylène en $C_1$ à $C_3$ et les autres restes $R^4$ à $R^{11}$ ainsi que, le cas échéant, le reste $R^2$ ou $R^3$ ont la définition indiquée conformément à la formule générale I.

6. Dérivés substitués de 1,5-dihydropyrrole-2-one suivant l'une des revendications 1 à 5, **caractérisés en ce que** le reste $R^4$ représente un reste alkyle en $C_1$ à $C_6$ et les autres restes $R^5$ à $R^{11}$ ont la définition indiquée conformément à la formule générale I.

7. Dérivés substitués de 1,5-dihydropyrrole-2-one suivant l'une des revendications 1 à 5, **caractérisés en ce que** le reste $R^4$ représente un reste aryle lié par l'intermédiaire d'un groupe alkylène en $C_1$ à $C_3$ et les autres restes $R^5$ à $R^{11}$ ont la définition indiquée conformément à la formule générale I.

8. Dérivés substitués de 1,5-dihydropyrrole-2-one suivant l'une des revendications 1 à 7, **caractérisés en ce que** les restes $R^5$ et $R^6$ représentent ensemble le groupe =O et les autres restes $R^7$ à $R^{11}$ ont la définition indiquée conformément à la formule générale I.

9. Dérivés substitués de 1,5-dihydropyrrole-2-one suivant l'une des revendications 1 à 7, **caractérisés en ce que** le reste $R^5$ et/ou le reste $R^6$ représentent un reste alkyle en $C_1$ à $C_6$ et les autres restes $R^7$ à $R^{11}$ ainsi que, le cas échéant, $R^5$ ou $R^6$ ont la définition indiquée conformément à la formule générale I.

10. Dérivés substitués de 1,5-dihydropyrrole-2-one suivant l'une des revendications 1 à 7, **caractérisés en ce que** le reste $R^5$ et/ou le reste $R^6$ représentent un reste aryle lié par l'intermédiaire d'un groupe alkylène en $C_1$ à $C_3$ et les autres restes $R^7$ à $R^{11}$ ainsi que, le cas échéant, $R^5$ ou $R^6$ ont la définition indiquée conformément à la formule générale I.

11. Dérivés substitués de 1,5-dihydropyrrole-2-one suivant l'une des revendications 1 à 10, **caractérisés en ce que** le reste $R^7$ représente un reste alkyle en $C_1$ à $C_6$ et les autres restes $R^8$ à $R^{11}$ ont la définition indiquée conformément à la formule générale I.

12. Dérivés substitués de 1,5-dihydropyrrole-2-one suivant l'une des revendications 1 à 10, **caractérisés en ce que** le reste $R^7$ représente un reste aryle lié par l'intermédiaire d'un groupe alkylène en $C_1$ à $C_3$ et les autres restes $R^8$ à $R^{11}$ ont la définition indiquée conformément à la formule générale I.

**13.** Dérivés substitués de 1,5-dihydropyrrole-2-one suivant l'une des revendications 1 à 12, **caractérisés en ce que** le reste $R^8$ représente un reste alkyle en $C_1$ à $C_6$ et les autres restes $R^9$ à $R^{11}$ ont la définition indiquée conformément à la formule générale I.

**14.** Dérivés substitués de 1,5-dihydropyrrole-2-one suivant l'une des revendications 1 à 12, **caractérisés en ce que** le reste $R^8$ représente un reste aryle lié par l'intermédiaire d'un groupe alkylène en $C_1$ à $C_3$ et les autres restes $R^9$ à $R^{11}$ ont la définition indiquée conformément à la formule générale I.

**15.** Dérivés substitués de 1,5-dihydropyrrole-2-one suivant l'une des revendications 1 à 14, **caractérisés en ce que** le reste $R^9$ représente un reste alkyle en $C_1$ à $C_6$ et les autres restes $R^{10}$ et $R^{11}$ ont la définition indiquée conformément à la formule générale I.

**16.** Dérivés substitués de 1-5-dihydropyrrole-2-one suivant l'une des revendications 1 à 14, **caractérisés en ce que** le reste $R^9$ représente un reste aryle lié par l'intermédiaire d'un groupe alkylène en $C_1$ à $C_3$ et les autres restes $R^{10}$ et $R^{11}$ ont la définition indiquée conformément à la formule générale I.

**17.** Dérivés substitués de 1,5-dihydropyrrole-2-one suivant l'une des revendications 1 à 16, **caractérisés en ce que** le reste $R^{10}$ représente un reste alkyle en $C_1$ à $C_6$ et le reste $R^{11}$ a la définition indiquée conformément à la formule générale I.

**18.** Dérivés substitués de 1,5-dihydropyrrole-2-one suivant l'une des revendications 1 à 16, **caractérisés en ce que** le reste $R^{10}$ représente un reste aryle lié par l'intermédiaire d'un groupe alkylène en $C_1$ à $C_3$ et le reste $R^{11}$ a la définition indiquée conformément à la formule générale I.

**19.** Dérivés substitués de 1,5-dihydropyrrole-2-one suivant l'une des revendications 1 à 18, **caractérisés en ce que** le reste $R^{11}$ représente un reste alkyle en $C_1$ à $C_6$.

**20.** Dérivés substitués de 1,5-dihydropyrrole-2-one suivant l'une des revendications 1 à 18, **caractérisés en ce que** le reste $R^{11}$ représente un reste aryle lié par l'intermédiaire d'un groupe alkylène en $C_1$ à $C_3$.

**21.** Dérivés substitués de 1,5-dihydropyrrole-2-one suivant la revendication 1, à savoir
5-benzylidène-4-méthoxy-3-nitroso-1,5-dihydropyrrole-2-one,
4-benzylamino-5-benzylidène-3-nitroso-1,5-dihydropyrrole-2-one,
5-benzylidène-4-hydroxy-3-nitroso-1,5-dihydropyrrole-2-one,
5-benzylidène-3-nitroso-4-phénylamino-1,5-dihydropyrrole-2-one,
5-benzylidène-4-méthylamino-3-nitroso-1,5-dihydropyrrole-2-one,
4-amino-5-benzylidène-3-nitroso-1,5-dihydropyrrole-2-one,
5-benzylidène-3-nitro-4-phénylamino-1,5-dihydropyrrole-2-one,
4-benzylamino-5-benzylidène-3-nitro-1,5-dihydropyrrole-2-one,
5-benzylidène-4-méthylamino-3-nitro-1,5-dihydropyrrole-2-one,
et
4-amino-5-benzylidène-3-nitro-1,5-dihydropyrrole-2-one.

**22.** Procédé de production de dérivés substitués de 1,5-dihydropyrrole-2-one de formule générale I suivant la revendication 1, **caractérisé en ce qu'**on transforme des acides tétramiques de formule générale II

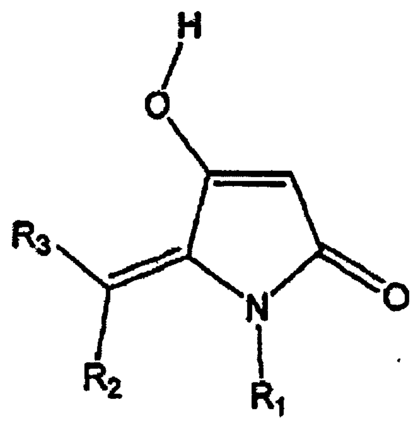

II

dans laquelle les restes $R^1$ à $R^3$ ont la définition indiquée conformément à la formule générale I, à basse température en solution, avantageusement en solution acide refroidie à la glace, notamment en solution acide, refroidie à la glace, d'acide acétique cristallisable, avec une solution aqueuse d'un nitrite alcalin, avantageusement de nitrite de sodium, en composés de formule générale III,

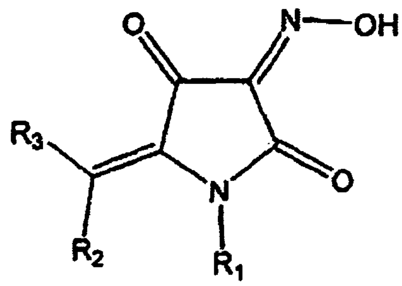

III

dans laquelle $R^1$ à $R^3$ ont la définition indiquée conformément à la formule générale I, et on purifie ces composés avantageusement par recristallisation, notamment dans l'éthanol, et on les isole,
puis on les transforme par alkylation avec des agents d'alkylation ou par réaction avec des chlorures d'acides, des bromures d'acides, des esters d'acide chlorocarbonique, des esters d'acide fluorocarbonique, des isocyanates et/ou des isothiocyanates dans des solvants non polaires et/ou dans des solvants aprotiques polaires et/ou dans des solvants protiques polaires, en composés de formule générale IV

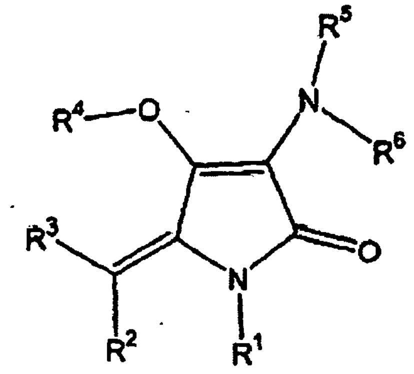

IV

IV

dans laquelle les restes $R^1$ à $R^4$ ont la définition indiquée conformément à la formule générale I et les restes $R^5$ et $R^6$ forment ensemble le groupe =O, et on purifie et isole ces composés par des modes opératoires usuels.

**23.** Procédé de production de dérivés substitués de 1,5-dihydropyrrole-2-one de formule générale I suivant la revendication 1, **caractérisé en ce qu'**on transforme les composés de formule générale IV selon la revendication 22, dans laquelle les restes $R^1$ à $R^4$ ont la définition indiquée selon la formule générale I et les restes $R^5$ et $R^6$ forment ensemble le groupe =O, par réaction avec des nucléophiles dans des solvants polaires en composés de formule générale V

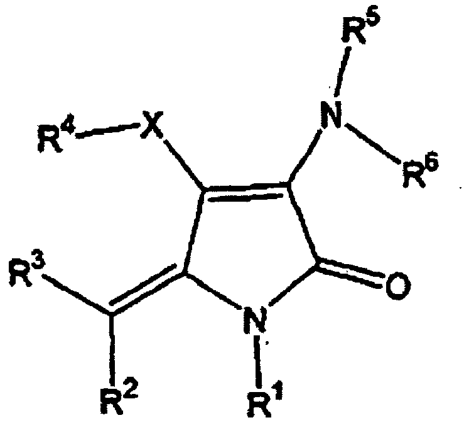

V

dans laquelle les restes $R^1$ à $R^4$ ainsi que X ont la définition indiquée conformément à la formule générale I et les restes $R^5$ et $R^6$ forment ensemble le groupe =O, et on purifie et isole ces composés par recristallisation, avantageusement dans le méthanol, l'éthanol et/ou l'isopropanol.

**24.** Procédé de production de dérivés substitués de 1,5-dihydropyrrole-2-one de formule générale I suivant la revendication 1, **caractérisé en ce qu'**on réduit les composés de formule générale V suivant la revendication 23 en composés de formule générale I dans laquelle $R^5$ et $R^6$ représentent chacun H et $R^1$ à $R^4$ ainsi que X ont la définition indiquée conformément à la formule générale I, et on purifie et isole ces composés par des modes opératoires usuels.

**25.** Procédé suivant la revendication 22, **caractérisé en ce qu'**on utilise comme agents d'alkylation des diazoalcanes, des sulfates de dialkyle, des halogénures d'alkyle ou un mélange de deux de ces composés, avantageusement des diazoalcanes en solution dans l'éther.

**26.** Procédé suivant la revendication 22, **caractérisé en ce qu'**on utilise comme solvants non polaires des éthers à chaîne ouverte et/ou des éthers cycliques, des hydrocarbures et/ou des hydrocarbures halogénés, comme solvants aprotiques polaires le diméthylformamide et/ou la N-méthylpyrrolidone et/ou comme solvant protique polaire le diméthylsulfoxyde.

**27.** Procédé suivant la revendication 23, **caractérisé en ce qu'**on utilise comme nucléophiles des amines primaires ou secondaires ou des alcoolates aliphatiques, aromatiques ou hétéroaromatiques ou des phénolates.

**28.** Procédé suivant la revendication 23, **caractérisé en ce qu'**on utilise comme solvant polaire le méthanol, l'éthanol et/ou l'isopropanol.

**29.** Procédé suivant la revendication 24, **caractérisé en ce qu'**on utilise pour la réduction le système Zn/acide acétique cristallisable, l'hydrure de lithium et d'aluminium, $BH_3$, le système $Na[BH_3CN]/TiCl_3$, des alcoolates de sodium ou l'hydrogène en présence de catalyseurs à base de métaux de transition.

**30.** Médicament contenant comme substance pharmaceutique active, au moins un dérivé substitué de 1,5-dihydropyrrole-2-one de formule générale Ia

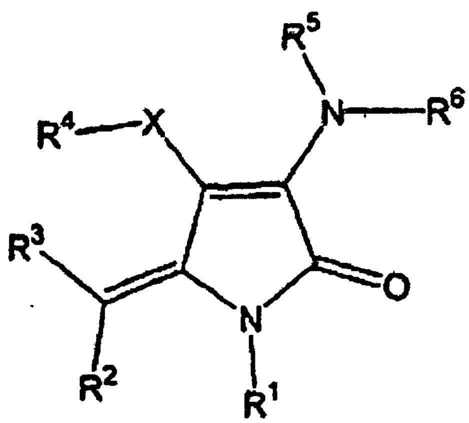

Ia

dans laquelle

X représente O, S ou $NR^7$,

le reste $R^1$ représente H, $OR^{11}$, $SR^{11}$, $COR^8$, $CSR^8$, $NR^9R^{10}$, $COOR^8$, $CONR^9R^{10}$, $CSNR^9R^{10}$, $COCOR^8$, un reste alkyle en $C_1$ à $C_{10}$, un reste aryle, un reste hétéroaryle, ou un reste aryle lié par l'intermédiaire d'un groupe alkylène en $C_1$ à $C_6$,

les restes $R^2$, $R^3$, identiques ou différents, représentent H, F, Cl, Br, $CF_3$, $OR^{11}$, $SR^{11}$, $NR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^4$ représente H, OH, $OR^{11}$, $SR^{11}$, $COR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

les restes $R^5$, $R^6$, identiques ou différents, représentent H, O, OH, $OR^{11}$, $SR^{11}$, $COR^8$, $CSR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$, ou bien les restes $R^5$ et $R^6$ représentent ensemble le groupe =O,

le reste $R^7$ représente H, $OR^{11}$, $SR^{11}$, $COR^8$, $COOR^8$, $COCOR^8$, $CONR^9R^{10}$, $CSNR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^8$ représente H, $OR^{11}$, $SR^{11}$, $NR^9R^{10}$, un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^9$ représente un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^{10}$ représente un reste alkyle en $C_1$ à $C_{10}$, un reste aryle, un reste hétéroaryle ou un reste aryle lié par l'intermédiaire d'un groupe alkylène en $C_1$ à $C_6$,

le reste $R^{11}$ représente un reste alkyle en $C_1$ à $C_{10}$, un reste aryle, un reste hétéroaryle ou un reste aryle lié par l'intermédiaire d'un groupe alkylène en $C_1$ à $C_6$, sous forme de son racémate, de ses énantiomères, de ses diastéréo-isomères ou d'une base correspondante ou d'un sel correspondant acceptable du point de vue physiologique, ainsi que, le cas échéant, d'autres substances actives et/ou des substances auxiliaires.

**31.** Médicament suivant la revendication 30, destiné à traiter/combattre des douleurs, des réactions inflammatoires et/ou allergiques, des dépressions, l'abus de drogues et/ou d'alcool, la gastrite, la diarrhée, l'incontinence urinaire, des maladies cardiovasculaires, des maladies des voies respiratoires, la toux, des maladies psychiques, et/ou l'épilepsie.

**32.** Médicament suivant la revendication 30, destiné au traitement/à la prophylaxie de la schizophrénie, de la maladie d'Alzheimer, de la maladie de Huntington, de la maladie de Parkinson, d'ischémies cérébrales, d'infarctus cérébraux, de psychoses causées par des taux élevés d'acides aminés, d'attaques d'apoplexie, d'oedèmes cérébraux, de l'hypoxie, de l'anoxie, de la démence liée au SIDA, de l'encéphalomyélite, du syndrome de Tourette, de l'asphyxie périnatale, des acouphènes et/ou destiné à un traitement anxiolytique.

**33.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de douleurs.

**34.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de réactions inflammatoires.

**35.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de réactions allergiques.

**36.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de dépressions.

**37.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'abus de drogues et/ou d'alcool.

**38.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la gastrite.

**39.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la diarrhée.

**40.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'incontinence urinaire.

**41.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique

pour la préparation d'un médicament destiné au traitement de maladies cardiovasculaires.

**42.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de maladies des voies respiratoires.

**43.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la toux.

**44.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de maladies psychiques.

**45.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'épilepsie.

**46.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la schizophrénie.

**47.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la maladie d'Alzheimer.

**48.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la maladie de Huntington.

**49.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la maladie de Parkinson.

**50.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement d'ischémies cérébrales.

**51.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement d'infarctus cérébraux.

**52.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de psychoses dues à des taux élevés d'acides aminés.

**53.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné à la prophylaxie d'attaques apoplexiques.

**54.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement d'oedèmes cérébraux.

**55.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'hypoxie.

**56.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'anoxie.

**57.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de la démence liée au SIDA.

**58.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'encéphalomyélite.

**59.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement du syndrome de Tourette.

**60.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement de l'asphyxie périnatale.

**61.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné à un traitement anxiolytique.

**62.** Utilisation d'au moins un dérivé substitué de 1,5-dihydropyrrole-2-one de formule générale Ia suivant la revendication 30 et/ou d'une base correspondante et/ou d'un sel correspondant acceptable du point de vue physiologique pour la préparation d'un médicament destiné au traitement des acouphènes.